# EUROPEAN PATENT SPECIFICATION

(11) **EP 1 311 677 B1**
(45) Date of publication and mention of the grant of the patent: **04.01.2006**
(21) Application number: 01976066.9
(22) Date of filing: 10.08.2001
(51) Int. Cl.: C12N 15/12, C12N 15/63, C12N 1/19, C12N 15/11, C12N 15/62, C07K 14/705, C07K 16/28, C12Q 1/68, G01N 33/68, A61K 31/7088, A61K 38/17, A61K 39/395, A61K 48/00

(54) **REGULATION OF HUMAN P2Y1-LIKE G PROTEIN-COUPLED RECEPTOR**
REGULIERUNG DES MENSCHLICHEN P2Y1-ÄHNLICHEN G-PROTEIN GEKOPPELTEN REZEPTORS
REGULATION DU RECEPTEUR COUPLE A LA PROTEINE G HUMAINE DU TYPE P2Y1

(30) Priority: 14.08.2000 US 224989 P
(43) Date of publication of application: 21.05.2003
(73) Proprietor: Bayer HealthCare AG, 51368 Leverkusen (DE)
(72) Inventor: RAMAKRISHNAN, Shyam, Brighton, MA 02135 (US)
(74) Representative: Ricker, Mathias
(86) International application number: PCT/EP2001/009243
(87) International publication number: WO 2002/014511

(56) References cited:
- WO-A-01/36471
- WO-A-01/36473
- WO-A-01/49847
- WO-A-01/57188
- WO-A-01/57190
- WO-A-01/66750
- WO-A-01/87937
- WO-A-01/87980
- DATABASE EMBL [Online] accession: AC026756, 24 March 2000 (2000-03-24) ABOLA A P ET AL: "Homo sapiens chromosome 13 clone RP11-286P8, complete sequence." XP002195810
- DATABASE EMBL [Online] accession: AW827323, 23 May 2000 (2000-05-23) NCI-CGAP: "hm31f01.x1 NCI_CGAP_Thy4 Homo sapiens cDNA clone IMAGE:3014233 3' similar to contains element MER17 repetitive element ;, mRNA sequence." XP002195811
- AYYANATHAN KASIRAJAN ET AL: "Cloning and chromosomal localization of the human P2Y1 purinoceptor." BIOCHEMICAL AND BIOPHYSICAL RESEARCH COMMUNICATIONS, vol. 218, no. 3, 1996, pages 783-788, XP002195807 ISSN: 0006-291X
- DATABASE EMBL [Online] accession: AJ006945, 16 October 1998 (1998-10-16) LEON C: "Human P2Y1 gene" XP002195815
- KUNAPULI SATYA P ET AL: "P2 receptor subtypes in the cardiovascular system." BIOCHEMICAL JOURNAL, vol. 336, no. 3, 15 December 1998 (1998-12-15), pages 513-523, XP002195808 ISSN: 0264-6021
- BARNARD ERIC A ET AL: "G protein-coupled receptors for ATP and other nucleotides: A new receptor family." TRENDS IN PHARMACOLOGICAL SCIENCES, vol. 15, no. 3, 1994, pages 67-70, XP002195809 ISSN: 0165-6147 cited in the application
- GROBBEN B. ET AL: 'Ecto-nucleotide pyrophosphatase modulates the purinoceptor-mediated signal transduction and is inhibited by purinoceptor antagonists.' BRITISH JOURNAL OF PHARMACOLOGY vol. 130, no. 1, May 2000, pages 139 - 145
- DIXON C.J.: 'Evidence that 2-methylthioATP and 2-methylthioADP are both agonists at the rat hepatocyte P2Y1 receptor.' BRITISH JOURNAL OF PHARMACOLOGY vol. 130, no. 3, June 2000, pages 664 - 668
- BANNER K.H.; PAGE C.P.: 'THEOPHYLLINE AND SELECTIVE PHOSPHODIESTERASE INHIBITORS AS ANTI-INFLAMMATORY DRUGS IN THE TREATMENT OF BRONCHIAL ASTHMA' EUROPEAN RESPIRATORY JOURNAL vol. 8, no. 6, 01 June 1995, MUNKSGAARD INTERNATIONAL PUBLISHERS, COPENHAGEN, DK, pages 996 - 1000, XP000575696

## Description

### TECHNICAL FIELD OF THE INVENTION

The invention relates to the area of G protein-coupled receptors. More particularly, it relates to the area of P2Y1-like G protein-coupled receptors and their regulation.

### BACKGROUND OF THE INVENTION

### G Protein-Coupled Receptors

Many medically significant biological processes are mediated by signal transduction pathways that involve G proteins (Lefkowitz, *Nature 351,* 353-354, 1991). The family of G protein-coupled receptors (GPCR) includes receptors for hormones, neurotransmitters, growth factors, and viruses. Specific examples of GPCRs include receptors for such diverse agents as dopamine, calcitonin, adrenergic hormones, endothelin, cAMP, adenosine, acetylcholine, serotonin, histamine, thrombin, kinin, follicle stimulating hormone, opsins, endothelial differentiation gene-1, rhodopsins, odorants, cytomegalovirus, G proteins themselves, effector proteins such as phospholipase C, adenyl cyclase, and phosphodiesterase, and actuator proteins such as protein kinase A and protein kinase C.

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, such as the β-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters *(see* Johnson *et al*., *Endoc. Rev. 10*, 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G protein connects the hormone receptor to adenylate cyclase. G protein exchanges GTP for bound GDP when activated by a hormone receptor. The GTP-carrying form then binds to activated adenylate cyclase. Hydrolysis of GTP to GDP, catalyzed by the G protein itself, returns the G protein to its basal, inactive form. Thus, the G protein serves a dual role, as an intermediate that relays the signal from receptor to effector, and as a clock that controls the duration of the signal.

Over the past 15 years, nearly 350 therapeutic agents targeting GPCRs have been successfully introduced onto the market. This indicates that these receptors have an established, proven history as therapeutic targets. Clearly, there is an on-going need for identification and characterization of further GPCRs which can play a role in preventing, ameliorating, or correcting dysfunctions or diseases.

### P2Y Receptors

Adenosine 5'-triphosphate (ATP) has many different physiological functions in the cell. For example, ATP is the energy source for many biochemical reactions, a precursor for ribonucleic acid (RNA) synthesis, the precursor for cyclic AMP synthesis, etc. ATP also functions as an extracellular messenger in neuronal and non-neuronal tissues. Extracellular ATP exerts its effects on these tissues by acting through membrane-associated purinoreceptors (Burnstock, G. Ann. NY Acad. Sci. (1990) 603:1-17). The purinoreceptors can be either ligand-gated ion channels (Bean, B. P. (1992) Trends Pharmac. Sci. 12:87-90; Bean, B. P. and Fried, D. D. (1990) Ion Channels 2:169-203) that are generally referred to as P2X receptors, (but also known as: purinergic channels, P2X R-channels, and ATP-gated channels) or G-protein-coupled (P2Y or P2V) receptors (Barnard, E. A. et al. (1994) Trends Pharmac. Sci. 15:67-70). See U.S. Patent 5,856,129.

P2Y1 receptors are abundant in brain (Filippov *et al., Br. J. Pharmacol. 129,* 1063-66, 2000) and are found in a variety of other locations, including vascular smooth muscle (Erlinge *et al., Biochem. Biophys. Res. Commun. 248,* 864-70, 2998), neonatal rat cardiac fibroblasts *(Zheng et al., Cardiovasc. Res. 37,* 718-28, 1998), and neonatal and adult cardiac myocytes *(Webb et al., J. Auton. Pharmacol. 16,* 303-07, 1996).

Because of the wide-spread distribution of GPCRs with diverse biological effects, including P2Y receptors, there is a need in the art to identify additional members of the GPCR family whose activity can be regulated to provide therapeutic effects. Ayyanathan Kasirajan et al. (Biochemical and biophysical research communications, vol. 218, no. 3, 1996, 783-788) disclose a human P2Y1 purinoceptor GPCR.

Banner K.H. and Page C.P. (European respiratory journal, vol. 8, no. 6, 1995, 996-1000) disclose the use of a screening method for the identification of compounds (e.g. theophylline and selective phosphodiesterase inhibitors) useful in the treatment of asthma.

### SUMMARY OF THE INVENTION

One embodiment of the invention is a method of screening for agents potentially useful for the treatment of asthma. A test compound is contacted with a P2Y1-like GPCR polypeptide comprising an amino acid sequence selected from the group consisting of:
amino acid sequences which are at least about 90% identical to the amino acid sequence shown in SEQ ID NO: 2; and
the amino acid sequence shown in SEQ ID NO: 2.

Binding between the test compound and the P2Y1-like GPCR polypeptide is detected. A test compound which binds to the P2Y1-like GPCR polypeptide is thereby identified as a potential agent for the treatment of asthma. The agent can work by decreasing the activity of the P2Y1-like GPCR.

Another embodiment of the invention is a further method of screening for agents which are potential agents for the treatment of asthma. A test compound is contacted with a P2Y1-like GPCR polypeptide comprising an amino acid sequence selected from the group consisting of:
amino acid sequences which are at least about 90% identical to the amino acid sequence shown in SEQ ID NO: 2; and
the amino acid sequence shown in SEQ ID NO: 2.

A P2Y1-like GPCR activity of the polypeptide is detected. A test compound which increases P2Y1-like GPCR activity of the polypeptide relative to P2Y1-like GPCR activity in the absence of the test compound is thereby identified as a potential agent for the treatment of asthma. A test compound which decreases P2Y1-like GPCR activity of the polypeptide relative to P2Y1-like GPCR activity in the absence of the test compound is thereby identified as a potential agent for the treatment of asthma.

Even another embodiment of the invention is a further method of screening for agents which are potentially useful for the treatment of asthma. A test compound is contacted with a P2Y1-like GPCR product of a polynucleotide which comprises a nucleotide sequence selected from the group consisting of:
nucleotide sequences which encode a protein with at least 90% identity to the amino acid sequence disclosed by SEQ ID NO: 2.

Binding of the test compound to the P2Y1-like GPCR product is detected. A test compound which binds to the P2Y1-like GPCR product is thereby identified as a potential agent for the treatment of asthma.

### BRIEF DESCRIPTION OF THE DRAWING

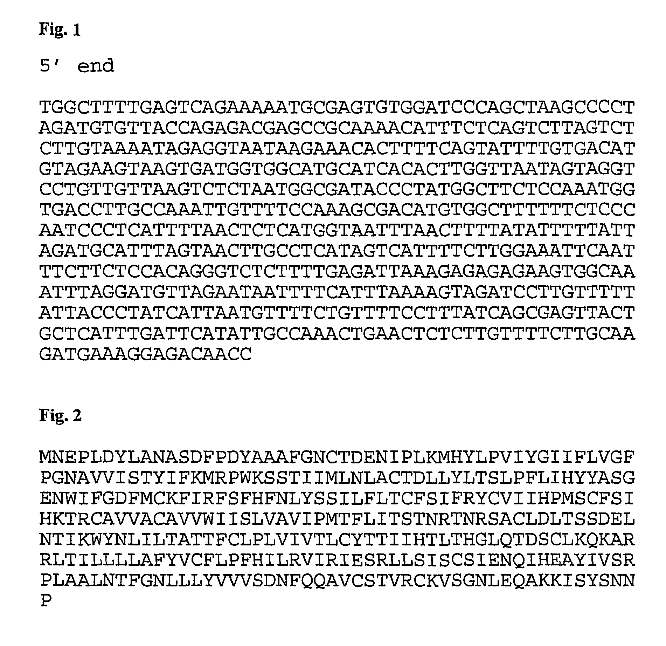

- Fig. 1: shows the DNA-sequence encoding a P2Y1-like GPCR polypeptide (SEQ ID NO: 1).
- Fig. 2: shows the amino acid sequence deduced from the DNA-sequence of Fig. 1 (SEQ ID NO:2).

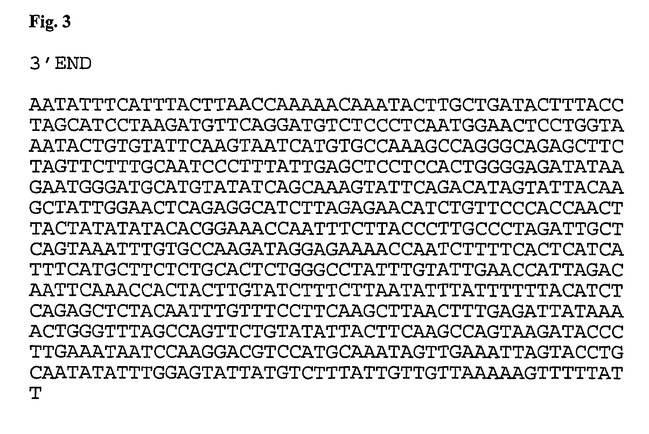

- Fig. 3: shows the DNA-sequence encoding a P2Y1-like GPCR polypeptide (SEQ ID NO:3).

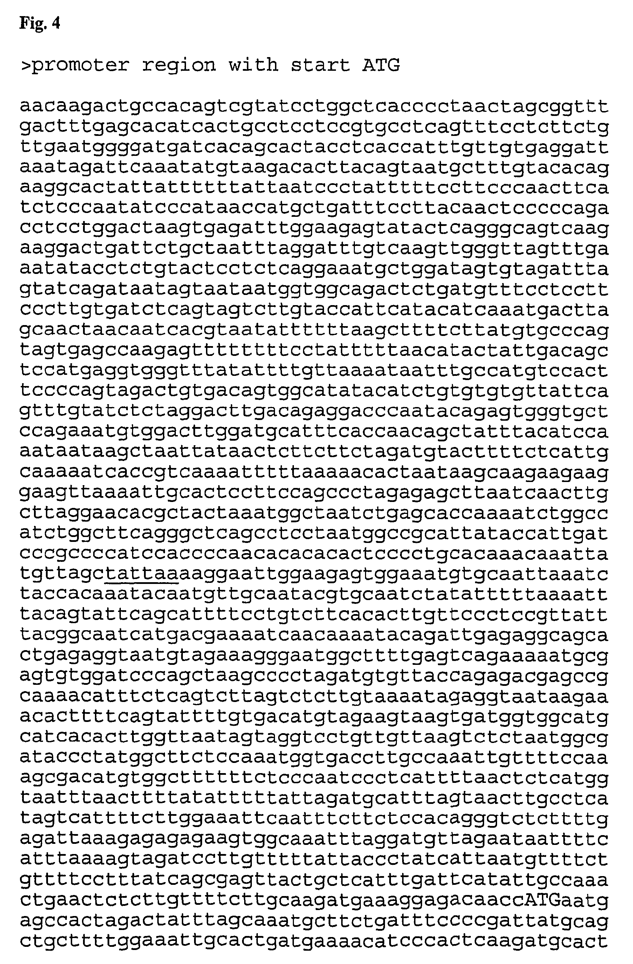

- Fig. 4: shows the DNA-sequence encoding a P2Y1-like GPCR polypeptide (SEQ ID NO:4).

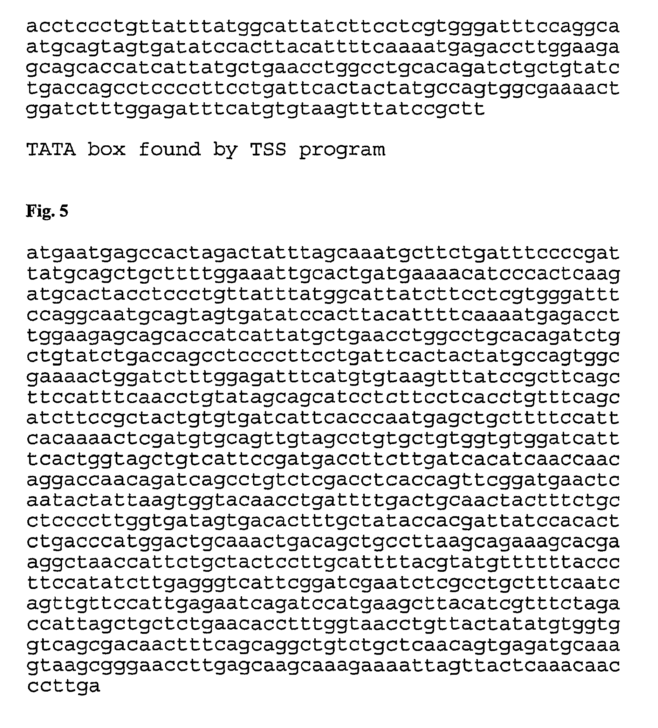

- Fig. 5: shows the DNA-sequence encoding a P2Y1-like GPCR polypeptide (SEQ ID NO:5).

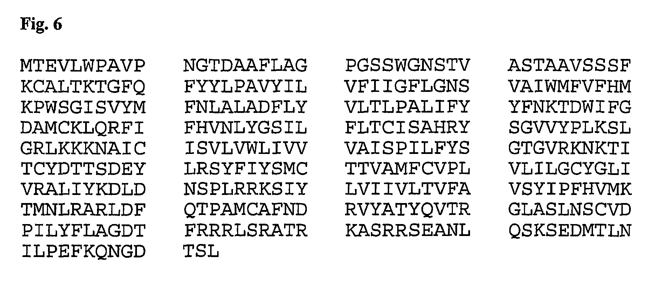

- Fig. 6: shows the amino acid sequence of the protein identified by SwissProt Accession No. P49650 (SEQ ID NO:6).

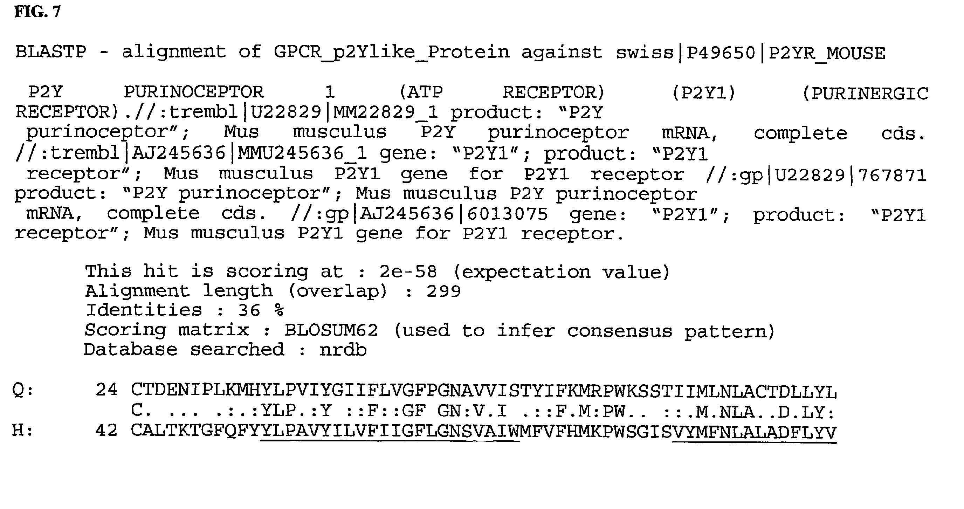

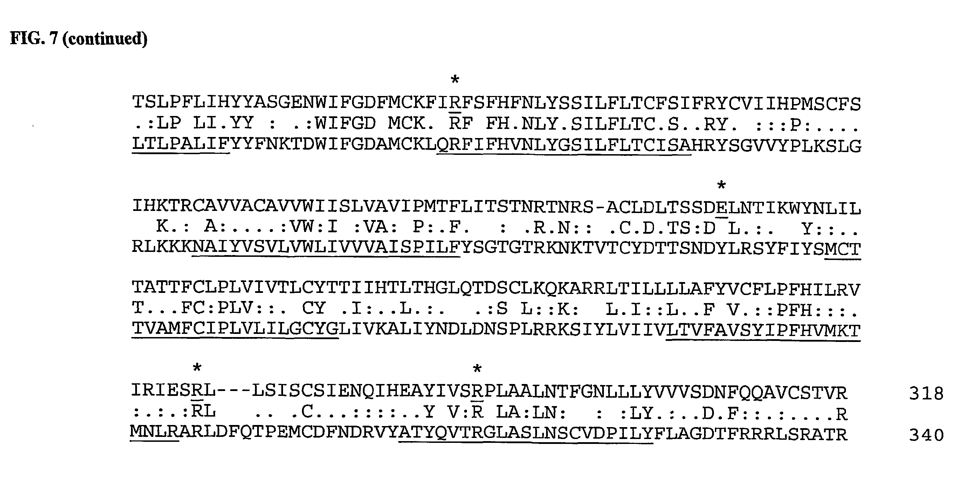

- Fig. 7: shows the BLASTP alignment of human P2Y1-like GPCR (SEQ ID NO:2) and the protein identified by SwissProt Accession No. P49650 (SEQ ID NO:6). Transmembrane domains on the sequence are highlited in bold and underlined. P2Y1 receptors having F226A, K280A, or Q307A mutations, do not bind the antagonist, 2'-deoxy-N6-methyladenosine 3', 5'-bisphosphate (MRS 2179), indicating that these residues are critical for the binding of the antagonist molecule. P2Y1-like GPCR is missing these three sites. Three sites which are critical for binding of ligands in human, two are conserved as shown by bold, underlined (no italics) residues on the query sequence. Mutations in each of these residues individually leads to loss of binding. Thus residues on the exofacial side of TM3 and TM7 are critical determinants of the ATP binding pocket. ATP may be docked in the receptor, both within the previously defined TM cleft and within two other regions of the receptor, termed meta-binding sites, defined by the extracellular loops. The first metabinding site is located outside of the TM bundle, between EL2 and EL3, and the second higher energy site is positioned immediately underneath EL2. Binding at both the principal TM binding site and the lower energy metabinding sites potentially affects the ligand potency. In meta-binding site I, the side chain of Glu (EL2) (Bold,Italic,Underlined, between TM domain 4 and 5) is within hydrogen-bonding distance(2.8 A) of the ribose O3', and Arg (EL3) (Bold,Italic,Underlined,between TM domain 6 and 7) coordinate both alpha- and beta-phosphates of the triphosphate chain.
- Fig. 8: shows the relative expression of human P2Y1-like G protein-coupled receptor in various human tissues and the neutrophil-like cell line HL60.
- Fig. 9: shows the relative expression of human P2Y1-like G protein-coupled receptor in respiratory cells and tissues.
- Fig.: 10 shows the relative expression of human P2Y1-like GPCR in various human tissues.

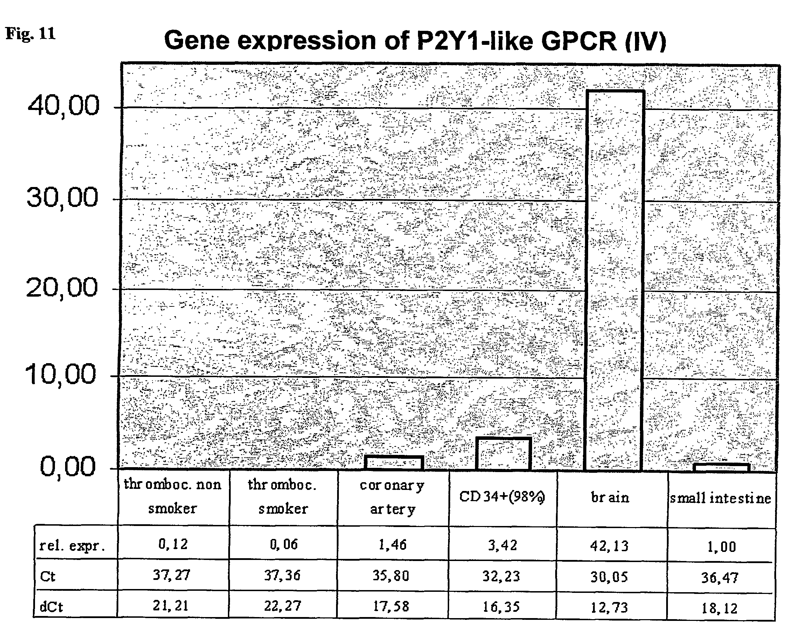

- Fig. 11: shows the relative expression of human P2Y1-like GPCR in human thrombocytes of non-smokers and smokers, in coronary arteries, in brain and in small instestine.

### DETAILED DESCRIPTION OF THE INVENTION

The invention relates to the use of an isolated polynucleotide encoding a P2Y1-like GPCR polypeptide and being selected from the group consisting of:
a polynucleotide encoding a P2Y1-like GPCR polypeptide comprising an amino acid sequence selected from the group consisting of amino acid sequences which are at least 90% identical to the amino acid sequence shown in SEQ ID NO: 2; and the amino acid sequence shown in SEQ ID NO: 2, and polypeptides encoded thereby for the screening for agents potentially useful for the treatment of asthma.

Disorders such as asthma can be treated by regulating human P2Y1-like GPCR. Human P2Y1-like GPCR also can be used to screen for human P2Y1-like GPCR agonists and antagonists.

### Polypeptides

P2Y1-like GPCR polypeptides according to the invention comprise at least 10, 12, 15, 20, 24, 30, 40, 50, 75, 100, 125, 150, 175, 200, 225, 250, 275, 300, 325, or 350 contiguous amino acids selected from the amino acid sequence shown in SEQ ID NO:2 or a biologically active variant of that sequence, as defined below. A P2Y1-like GPCR polypeptide of the invention therefore can be a portion of a P2Y1-like GPCR, a full-length P2Y1-like GPCR, or a fusion protein comprising all or a portion of a P2Y1-like GPCR.

### Biologically Active Variants

P2Y1-like GPCR polypeptide variants which are biologically active, i.e., retain the ability to bind a ligand to produce a biological effect, such as cyclic AMP formation, mobilization of intracellular calcium, or phosphoinositide metabolism, also are P2Y1-like GPCR polypeptides. Preferably, naturally or non-naturally occurring P2Y1-like GPCR polypeptide variants have amino acid sequences which are at least 90, 96, or 98% identical to an amino acid sequence shown in SEQ ID NO:2 or a fragment thereof. Percent identity between a putative P2Y1-like GPCR polypeptide variant and an amino acid sequence of SEQ ID NO:2 is determined using the Blast2 alignment program.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a P2Y1-like GPCR polypeptide can be found using computer programs well known in the art, such as DNASTAR software. Whether an amino acid change results in a biologically active P2Y1-like GPCR polypeptide can readily be determined by assaying for binding to a ligand or by conducting a functional assay, as described for example, in the specific Examples, below.

### Polynucleotides

A P2Y1-like GPCR polynucleotide can be single- or double-stranded and comprises a coding sequence or the complement of a coding sequence for a P2Y1-like GPCR polypeptide. A nucleotide sequence encoding SEQ ID NO:2 is shown in SEQ ID NO:5. The 5' and 3' ends of the human P2Y1-like GPCR gene are shown in SEQ ID NOS:1 and 3, respectively. The promoter region with the start ATG is shown in SEQ LD NO:4.

### Obtaining Polypeptides

P2Y1-like GPCR polypeptides can be obtained, for example, by purification from cells, by expression of P2Y1-like GPCR polynucleotides, or by direct chemical synthesis.

### Protein Purification

P2Y1-like GPCR polypeptides can be purified from any cell which expresses the receptor, including host cells which have been transfected with P2Y1-like GPCR polynucleotides which express such polypeptides. A purified P2Y1-like GPCR polypeptide is separated from other compounds which normally associate with the P2Y1-like GPCR polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

A P2Y1-like GPCR polypeptide can be conveniently isolated as a complex with its associated G protein, as described in the specific examples, below. A preparation of purified P2Y1-like GPCR polypeptides is at least 80% pure; preferably, the preparations are 90%, 95%, or 99% pure. Purity of the preparations can be assessed by any means known in the art, such as SDS-polyacrylamide gel electrophoresis.

### Expression of Polynucleotides

To express a P2Y1-like GPCR polypeptide, a P2Y1-like GPCR polynucleotide can be inserted into an expression vector which contains the necessary elements for the transcription and translation of the inserted coding sequence. Methods which are well known to those skilled in the art can be used to construct expression vectors containing sequences encoding P2Y1-like GPCR polypeptides and appropriate transcriptional and translational control elements. These methods include *in vitro* recombinant DNA techniques, synthetic techniques, and *in vivo* genetic recombination. Such techniques are described, for example, in Sambrook *et al*. (1989) and in Ausubel *et al*., CURRENT PROTOCOLS IN MOLECULAR BIOLOGY, John Wiley & Sons, New York, N.Y., 1989.

A variety of expression vector/host systems can be utilized to contain and express sequences encoding a P2Y1-like GPCR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g*., baculovirus), plant cell systems transformed with virus expression vectors *(e.g.,* cauliflower mosaic virus, CaMV; tobacco mosaic virus, TMV) or with bacterial expression vectors (*e.g*., Ti or pBR322 plasmids), or animal cell systems.

The control elements or regulatory sequences are those non-translated regions of the vector -- enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses *(e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a P2Y1-like GPCR polypeptide, vectors based on SV40 or EBV can be used with an appropriate selectable marker.

### Bacterial and Yeast Expression Systems

In bacterial systems, a number of expression vectors can be selected depending upon the use intended for the P2Y1-like GPCR polypeptide. For example, when a large quantity of a P2Y1-like GPCR polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the P2Y1-like GPCR polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem. 264,* 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al.* (1989) and Grant *et al., Methods Enzymol. 153, 516-544,* 1987.

### Plant and Insect Expression Systems

If plant expression vectors are used, the expression of sequences encoding P2Y1-like GPCR polypeptides can be driven by any of a number of promoters. For example, viral promoters such as the 35S and 19S promoters of CaMV can be used alone or in combination with the omega leader sequence from TMV (Takamatsu, *EMBO J. 6,* 307-311, 1987). Alternatively, plant promoters such as the small subunit of RUBISCO or heat shock promoters can be used (Coruzzi *et al., EMBO J. 3,* 1671-1680, 1984; Broglie *et al., Science 224,* 838-843, 1984; Winter *et al., Results Probl. Cell Differ. 17*, 85-105, 1991). These constructs can be introduced into plant cells by direct DNA transformation or by pathogen-mediated transfection. Such techniques are described in a number of generally available reviews (*e.g*., Hobbs or Murray, in MCGRAW HILL YEARBOOK OF SCIENCE AND TECHNOLOGY, McGraw Hill, New York, N.Y., pp. 191-196, 1992).

An insect system also can be used to express a P2Y1-like GPCR polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding P2Y1-like GPCR polypeptides can be cloned into a non-essential region of the virus, such as the polyhedrin gene, and placed under control of the polyhedrin promoter. Successful insertion of P2Y1-like GPCR polypeptides will render the polyhedrin gene inactive and produce recombinant virus lacking coat protein. The recombinant viruses can then be used to infect *S. frugiperda cells* or *Trichoplusia* larvae in which P2Y1-like GPCR polypeptides can be expressed (Engelhard *et al., Proc. Nat. Acad. Sci. 91,* 3224-3227, 1994).

### Mammalian Expression Systems

A number of viral-based expression systems can be used to express P2Y1-like GPCR polypeptides in mammalian host cells. For example, if an adenovirus is used as an expression vector, sequences encoding P2Y1-like GPCR polypeptides can be ligated into an adenovirus transcription/translation complex comprising the late promoter and tripartite leader sequence. Insertion in a non-essential E1 or E3 region of the viral genome can be used to obtain a viable virus which is capable of expressing a P2Y1-like GPCR polypeptide in infected host cells (Logan & Shenk, *Proc. Natl. Acad. Sci. 81,* 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding P2Y1-like GPCR polypeptides. Such signals include the ATG initiation codon and adjacent sequences. In cases where sequences encoding a P2Y1-like GPCR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al., Results Probl. Cell Differ. 20,* 125-162, 1994).

### Host Cells

A host cell strain can be chosen for its ability to modulate the expression of the inserted sequences or to process the expressed P2Y1-like GPCR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g*., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express P2Y1-like GPCR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced P2Y1-like GPCR sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines. These include, but are not limited to, the herpes simplex virus thymidine kinase (Wigler *et al., Cell 11*, 223-32, 1977) and adenine phosphoribosyltransferase (Lowy *et al., Cell 22*, 817-23, 1980) genes which can be employed in *tk*^{*-*} or *aprt*^{*-*} cells, respectively. Also, antimetabolite, antibiotic, or herbicide resistance can be used as the basis for selection. For example, *dhfr* confers resistance to methotrexate (Wigler *et al., Proc. Natl. Acad. Sci. 77,* 3567-70, 1980), *npt* confers resistance to the aminoglycosides, neomycin and G-418 (Colbere-Garapin *et al., J. Mol. Biol*. *150*, 1-14, 1981), and *als* and *pat* confer resistance to chlorsulfuron and phosphinotricin acetyltransferase, respectively (Murray, 1992, *supra*). Additional selectable genes have been described. For example, *trpB* allows cells to utilize indole in place of tryptophan, or *hisD,* which allows cells to utilize histinol in place of histidine (Hartman & Mulligan, *Proc. Natl. Acad*. *Sci. 85,* 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al*., *Methods Mol. Biol. 55*, 121-131, 1995).

### Detecting Expression of Polypeptides

Although the presence of marker gene expression suggests that the P2Y1-like GPCR polynucleotide is also present, its presence and expression may need to be confirmed. For example, if a sequence encoding a P2Y1-like GPCR polypeptide is inserted within a marker gene sequence, transformed cells containing sequences which encode a P2Y1-like GPCR polypeptide can be identified by the absence of marker gene function. Alternatively, a marker gene can be placed in tandem with a sequence encoding a P2Y1-like GPCR polypeptide under the control of a single promoter. Expression of the marker gene in response to induction or selection usually indicates expression of the P2Y1-like GPCR polynucleotide.

Alternatively, host cells which contain a P2Y1-like GPCR polynucleotide and which express a P2Y1-like GPCR polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding a P2Y1-like GPCR polypeptide can be detected by DNA-DNA or DNA-RNA hybridization or amplification using probes or fragments or fragments of polynucleotides encoding a P2Y1-like GPCR polypeptide. Nucleic acid amplification-based assays involve the use of oligonucleotides selected from sequences encoding a P2Y1-like GPCR polypeptide to detect transformants which contain a P2Y1-like GPCR polynucleotide.

A variety of protocols for detecting and measuring the expression of a P2Y1-like GPCR polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a P2Y1-like GPCR polypeptide can be used, or a competitive binding assay can be employed. These and other assays are described in Hampton *et al*., SEROLOGICAL METHODS: A LABORATORY MANUAL, APS Press, St. Paul, Minn., 1990) and Maddox *et al., J. Exp. Med. 158*, 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding P2Y1-like GPCR polypeptides include oligolabeling, nick translation, end-labeling, or PCR amplification using a labeled nucleotide. Alternatively, sequences encoding a P2Y1-like GPCR polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

### Expression and Purification of Polypeptides

Host cells transformed with nucleotide sequences encoding a P2Y1-like GPCR polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode P2Y1-like GPCR polypeptides can be designed to contain signal sequences which direct secretion of soluble P2Y1-like GPCR polypeptides through a prokaryotic or eukaryotic cell membrane or which direct the membrane insertion of membrane-bound P2Y1-like GPCR polypeptide.

As discussed above, other constructions can be used to join a sequence encoding a P2Y1-like GPCR polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the P2Y1-like GPCR polypeptide also can be used to facilitate purification. One such expression vector provides for expression of a fusion protein containing a P2Y1-like GPCR polypeptide and 6 histidine residues preceding a thioredoxin or an enterokinase cleavage site. The histidine residues facilitate purification by IMAC (immobilized metal ion affinity chromatography, as described in Porath *et al., Prot. Exp. Purif. 3,* 263-281, 1992), while the enterokinase cleavage site provides a means for purifying the P2Y1-like GPCR polypeptide from the fusion protein. Vectors which contain fusion proteins are disclosed in Kroll *et al., DNA Cell Biol. 12*, 441-453, 1993.

### Chemical Synthesis

Sequences encoding a P2Y1-like GPCR polypeptide can be synthesized, in whole or in part, using chemical methods well known in the art (see Caruthers *et al., Nucl. Acids Res. Symp. Ser.* 215-223, 1980; Hom *et al. Nucl. Acids Res. Symp. Ser.* 225-232, 1980). Alternatively, a P2Y1-like GPCR polypeptide itself can be produced using chemical methods to synthesize its amino acid sequence, such as by direct peptide synthesis using solid-phase techniques (Merrifield, *J. Am. Chem*. *Soc. 85*, 2149-2154, 1963; Roberge *et al., Science 269,* 202-204, 1995). Protein synthesis can be performed using manual techniques or by automation. Automated synthesis can be achieved, for example, using Applied Biosystems 431A Peptide Synthesizer (Perkin Elmer). Optionally, fragments of P2Y1-like GPCR polypeptides can be separately synthesized and combined using chemical methods to produce a full-length molecule.

The newly synthesized peptide can be substantially purified by preparative high performance liquid chromatography (*e*.*g*., Creighton, PROTEINS: STRUCTURES AND MOLECULAR PRINCIPLES, WH Freeman and Co., New York, N.Y., 1983). The composition of a synthetic P2Y1-like GPCR polypeptide can be confirmed by amino acid analysis or sequencing *(e.g.,* the Edman degradation procedure; *see* Creighton, *supra*). Additionally, any portion of the amino acid sequence of the P2Y1-like GPCR polypeptide can be altered during direct synthesis and/or combined using chemical methods with sequences from other proteins to produce a variant polypeptide or a fusion protein.

### Production of Altered Polypeptides

As will be understood by those of skill in the art, it may be advantageous to produce P2Y1-like GPCR polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter P2Y1-like GPCR polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

### Differentially Expressed Genes

Described herein are methods for the identification of genes whose products interact with human P2Y1-like G protein-coupled receptor. Such genes may represent genes that are differentially expressed in disorders including, but not limited to, CNS disorders, cardiovascular disorders, asthma, osteoporosis, diabetes, and COPD.

Further, such genes may represent genes that are differentially regulated in response to manipulations relevant to the progression or treatment of such diseases. Additionally, such genes may have a temporally modulated expression, increased or decreased at different stages of tissue or organism development. A differentially expressed gene may also have its expression modulated under control versus experimental conditions. In addition, the human P2Y1-like G protein-coupled receptor gene or gene product may itself be tested for differential expression.

The degree to which expression differs in a normal versus a diseased state need only be large enough to be visualized via standard characterization techniques such as differential display techniques. Other such standard characterization techniques by which expression differences may be visualized include but are not limited to, quantitative RT (reverse transcriptase), PCR, and Northern analysis.

### Identification of Differentially Expressed Genes

To identify differentially expressed genes total RNA or, preferably, mRNA is isolated from tissues of interest. For example, RNA samples are obtained from tissues of experimental subjects and from corresponding tissues of control subjects. Any RNA isolation technique that does not select against the isolation of mRNA may be utilized for the purification of such RNA samples. See, for example, Ausubel *et al*., ed., CURRENT PROTOCOLS IN MOLECULAR BIOLOGY, John Wiley & Sons, Inc. New York, 1987-1993. Large numbers of tissue samples may readily be processed using techniques well known to those of skill in the art, such as, for example, the single-step RNA isolation process of Chomczynski, U.S. Patent 4,843,155.

Transcripts within the collected RNA samples that represent RNA produced by differentially expressed genes are identified by methods well known to those of skill in the art. They include, for example, differential screening (Tedder *et al., Proc. Natl. Acad. Sci. U.S.A. 85,* 208-12, 1988), subtractive hybridization (Hedrick *et al., Nature 308,* 149-53; Lee *et al., Proc. Natl. Acad. Sci. U.S.A. 88*, 2825, 1984), and differential display (Liang & Pardee, *Science 257,* 967-71, 1992; U.S. Patent 5,262,311), and microarrays.

The differential expression information may itself suggest relevant methods for the treatment of disorders involving the human P2Y1-like G protein-coupled receptor. For example, treatment may include a modulation of expression of the differentially expressed genes and/or the gene encoding the human P2Y1-like G protein-coupled receptor. The differential expression information may indicate whether the expression or activity of the differentially expressed gene or gene product or the human P2Y1-like G protein-coupled receptor gene or gene product are up-regulated or down-regulated.

### Screening Methods

The invention provides assays for screening test compounds which bind to or modulate the activity of a P2Y1-like GPCR polypeptide or a P2Y1-like GPCR polynucleotide. A test compound preferably binds to a P2Y1-like GPCR polypeptide or polynucleotide. More preferably, a test compound decreases or increases a biological effect mediated via human P2Y1-like GPCR by at least about 10, preferably about 50, more preferably about 75, 90, or 100% relative to the absence of the test compound.

### Test Compounds

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des.* 12, 145, 1997.

Methods for the synthesis of molecular libraries are well known in the art *(see,* for example, DeWitt *et al., Proc. Natl. Acad. Sci. U.S.A. 90*, 6909, 1993; Erb *et al. Proc. Natl. Acad Sci. U.S.A. 91,* 11422, 1994; Zuckermann *et al., J. Med. Chem. 37*, 2678, 1994; Cho *et al., Science 261*, 1303, 1993; Carell *et al., Angew. Chem. Int. Ed. Engl. 33*, 2059, 1994; Carell *et al., Angew. Chem. Int. Ed. Engl. 33*, 2061; Gallop *et al., J. Med. Chem. 37,* 1233, 1994). Libraries of compounds can be presented in solution *(see, e.g.,* Houghten, *BioTechniques 13,* 412-421, 1992), or on beads (Lam, *Nature 354*, 82-84, 1991), chips (Fodor, *Nature 364,* 555-556, 1993), bacteria or spores (Ladner, U.S. Patent 5,223,409), plasmids (Cull *et al*., *Proc. Natl. Acad. Sci. U.S.A.* 89, 1865-1869, 1992), or phage (Scott & Smith, *Science 249,* 386-390, 1990*;* Devlin, *Science 249,* 404-406, 1990); Cwirla *et al., Proc. Natl. Acad. Sci. 97,* 6378-6382, 1990; Felici, *J. Mol*. *Biol. 222,* 301-310, 1991; and Ladner, U.S. Patent 5,223,409).

### High Throughput Screening

Test compounds can be screened for the ability to bind to P2Y1-like GPCR polypeptides or polynucleotides or to affect P2Y1-like GPCR activity or P2Y1-like GPCR gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 µl. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al., Proc. Natl. Acad. Sci. U.S.A. 19,* 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by Salmon *et al., Molecular Diversity 2,* 57-63 (1996). In this example, combinatorial libraries were screened for compounds that had cytotoxic effects on cancer cells growing in agar.

Another high throughput screening method is described in Beutel *et al*., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

### Binding Assays

For binding assays, the test compound is preferably a small molecule which binds to and occupies the active site of the P2Y1-like GPCR polypeptide, thereby making the ligand binding site inaccessible to substrate such that normal biological activity is prevented. Examples of such small molecules include, but are not limited to, small peptides or peptide-like molecules. Potential ligands which may bind to a polypeptide of the invention include, but are not limited to, the natural ligands of known GPCRs and analogues or derivatives thereof. Natural ligands of GPCRs include adrenomedullin, amylin, calcitonin gene related protein (CGRP), calcitonin, anandamide, serotonin, histamine, adrenalin, noradrenalin, platelet activating factor, thrombin, C5a, bradykinin, and chemokines.

In binding assays, either the test compound or the P2Y1-like GPCR polypeptide can comprise a detectable label, such as a fluorescent, radioisotopic, chemiluminescent, or enzymatic label, such as horseradish peroxidase, alkaline phosphatase, or luciferase. Detection of a test compound which is bound to the P2Y1-like GPCR polypeptide can then be accomplished, for example, by direct counting of radioemmission, by scintillation counting, or by determining conversion of an appropriate substrate to a detectable product.

Alternatively, binding of a test compound to a P2Y1-like GPCR polypeptide can be determined without labeling either of the interactants. For example, a microphysiometer can be used to detect binding of a test compound with a P2Y1-like GPCR polypeptide. A microphysiometer (*e.g*., Cytosensor™) is an analytical instrument that measures the rate at which a cell acidifies its environment using a light-addressable potentiometric sensor (LAPS). Changes in this acidification rate can be used as an indicator of the interaction between a test compound and a P2Y1-like GPCR polypeptide (McConnell *et al., Science 257,* 1906-1912, 1992).

Determining the ability of a test compound to bind to a P2Y1-like GPCR polypeptide also can be accomplished using a technology such as real-time Bimolecular Interaction Analysis (BIA) (Sjolander & Urbaniczky, *Anal. Chem. 63,* 2338-2345, 1991, and Szabo *et al., Curr. Opin. Struct. Biol. 5*, 699-705, 1995). BIA is a technology for studying biospecific interactions in real time, without labeling any of the interactants (*e.g*., BIAcore™). Changes in the optical phenomenon surface plasmon resonance (SPR) can be used as an indication of real-time reactions between biological molecules.

In yet another aspect of the invention, a P2Y1-like GPCR polypeptide can be used as a "bait protein" in a two-hybrid assay or three-hybrid assay (see, e.g., U.S. Patent 5,283,317; Zervos *et al., Cell 72,* 223-232, 1993; Madura *et al., J. Biol. Chem. 268,* 12046-12054, 1993; Bartel *et al., BioTechniques 14,* 920-924, 1993; Iwabuchi *et al., Oncogene 8,* 1693-1696, 1993; and Brent W094/10300), to identify other proteins which bind to or interact with the P2Y1-like GPCR polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a P2Y1-like GPCR polypeptide can be fused to a polynucleotide encoding the DNA binding domain of a known transcription factor (*e.g*., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g*., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the P2Y1-like GPCR polypeptide.

It may be desirable to immobilize either the P2Y1-like GPCR polypeptide (or polynucleotide) or the test compound to facilitate separation of bound from unbound forms of one or both of the interactants, as well as to accommodate automation of the assay. Thus, either the P2Y1-like GPCR polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the P2Y1-like GPCR polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a P2Y1-like GPCR polypeptide (or polynucleotide) can be accomplished in any vessel suitable for containing the reactants. Examples of such vessels include microtiter plates, test tubes, and microcentrifuge tubes.

In one embodiment, the P2Y1-like GPCR polypeptide is a fusion protein comprising a domain that allows the P2Y1-like GPCR polypeptide to be bound to a solid support. For example, glutathione-S-transferase fusion proteins can be adsorbed onto glutathione sepharose beads (Sigma Chemical) or glutathione derivatized microtiter plates, which are then combined with the test compound or the test compound and the non-adsorbed P2Y1-like GPCR polypeptide; the mixture is then incubated under conditions conducive to complex formation (*e.g*., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a P2Y1-like GPCR polypeptide (or polynucleotide) or a test compound can be immobilized utilizing conjugation of biotin and streptavidin. Biotinylated P2Y1-like GPCR polypeptides (or polynucleotides) or test compounds can be prepared from biotin-NHS(N-hydroxysuccinimide) using techniques well known in the art *(e.g.,* biotinylation kit, Pierce Chemicals, Rockford, Ill.) and immobilized in the wells of streptavidin-coated 96 well plates (Pierce Chemical). Alternatively, antibodies which specifically bind to a P2Y1-like GPCR polypeptide, polynucleotide, or a test compound, but which do not interfere with a desired binding site, such as the active site of the P2Y1-like GPCR polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the P2Y1-like GPCR polypeptide or test compound, enzyme-linked assays which rely on detecting an activity of the P2Y1-like GPCR polypeptide, and SDS gel electrophoresis under non-reducing conditions.

Screening for test compounds which bind to a P2Y1-like GPCR polypeptide or polynucleotide also can be carried out in an intact cell. Any cell which comprises a P2Y1-like GPCR polypeptide or polynucleotide can be used in a cell-based assay system. A P2Y1-like GPCR polynucleotide can be naturally occurring in the cell or can be introduced using techniques such as those described above. Binding of the test compound to a P2Y1-like GPCR polypeptide or polynucleotide is determined as described above.

### Functional Assays

Test compounds can be tested for the ability to increase or decrease a biological effect of a P2Y1-like GPCR polypeptide. Such biological effects can be determined using the functional assays described in the specific examples, below. Functional assays can be carried out after contacting either a purified P2Y1-like GPCR polypeptide, a cell membrane preparation, or an intact cell with a test compound. A test compound which decreases a functional activity of a P2Y1-like GPCR by at least about 10, preferably about 50, more preferably about 75, 90, or 100% is identified as a potential agent for decreasing P2Y1-like GPCR activity. A test compound which increases P2Y1-like GPCR activity by at least about 10, preferably about 50, more preferably about 75, 90, or 100% is identified as a potential agent for increasing P2Y1-like GPCR activity.

One such screening procedure involves the use of melanophores which are transfected to express a P2Y1-like GPCR polypeptide. Such a screening technique is described in WO 92/01810 published Feb. 6, 1992. Thus, for example, such an assay may be employed for screening for a compound which inhibits activation of the receptor polypeptide by contacting the melanophore cells which comprise the receptor with both a receptor ligand and a test compound to be screened. Inhibition of the signal generated by the ligand indicates that a test compound is a potential antagonist for the receptor, i.e., inhibits activation of the receptor. The screen may be employed for identifying a test compound which activates the receptor by contacting such cells with compounds to be screened and determining whether each test compound generates a signal, *i.e.,* activates the receptor.

Other screening techniques include the use of cells which express a human P2Y1-like GPCR polypeptide (for example, transfected CHO cells) in a system which measures extracellular pH changes caused by receptor activation *(see, e.g., Science 246,* 181-296, 1989). For example, test compounds may be contacted with a cell which expresses a human P2Y1-like GPCR polypeptide and a second messenger response, e.g., signal transduction or pH changes, can be measured to determine whether the test compound activates or inhibits the receptor.

Another such screening technique involves introducing RNA encoding a human P2Y1-like GPCR polypeptide into *Xenopus* oocytes to transiently express the receptor. The transfected oocytes can then be contacted with the receptor ligand and a test compound to be screened, followed by detection of inhibition or activation of a calcium signal in the case of screening for test compounds which are thought to inhibit activation of the receptor.

Another screening technique involves expressing a human P2Y1-like GPCR polypeptide in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Details of functional assays such as those described above are provided in the specific examples, below.

### Therapeutic Indications and Methods

GPCRs are ubiquitous in the mammalian host and are responsible for many biological functions, including many pathologies. Accordingly, it is desirable to find compounds and drugs which stimulate a GPCR on the one hand and which can inhibit the function of a GPCR on the other hand. For example, compounds which activate a GPCR may be employed for therapeutic purposes, such as the treatment of asthma.
In general, compounds which inhibit activation of a GPCR can be used for a variety of therapeutic purposes, for example, for the treatment of asthma

Asthma can be treated by regulating human P2Y1-like GPCR. Asthma is though to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness caused by a decreased control of airway caliber, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to the tissue destruction related to the disorder. Other resident cells, such as smooth muscle cells, lung epithelial cells, mucus-producing cells, and nerve cells may also be abnormal in individuals with asthma and may contribute to the pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually make asthma a chronic disabling disorder requiring long-term management.

Despite recent important advances in our understanding of the pathophysiology of asthma, the disease appears to be increasing in prevalence and severity (Gergen and Weiss, *Am. Rev. Respir. Dis. 146,* 823-24, 1992). It is estimated that 30-40% of the population suffer with atopic allergy, and 15% of children and 5% of adults in the population suffer from asthma (Gergen and Weiss, 1992). Thus, an enormous burden is placed on our health care resources. However, both diagnosis and treatment of asthma are difficult. The severity of lung tissue inflammation is not easy to measure and the symptoms of the disease are often indistinguishable from those of respiratory infections, chronic respiratory inflammatory disorders, allergic rhinitis, or other respiratory disorders. Often, the inciting allergen cannot be determined, making removal of the causative environmental agent difficult. Current pharmacological treatments suffer their own set of disadvantages. Commonly used therapeutic agents, such as beta agonists, can act as symptom relievers to transiently improve pulmonary function, but do not affect the underlying inflammation. Agents that can reduce the underlying inflammation, such as anti-inflammatory steroids, can have major drawbacks that range from immunosuppression to bone loss (Goodman and Gilman's THE PHARMACOLOGIC BASIS OF THERAPEUTICS, Seventh Edition, MacMillan Publishing Company, NY, USA, 1985). In addition, many of the present therapies, such as inhaled corticosteroids, are short-lasting, inconvenient to use, and must be used often on a regular basis, in some cases for life, making failure of patients to comply with the treatment a major problem and thereby reducing their effectiveness as a treatment.

Because of the problems associated with conventional therapies, alternative treatment strategies have been evaluated. Glycophorin A (Chu and Sharom, *Cell. Immunol*. *145,* 223-39, 1992), cyclosporin (Alexander *et al., Lancet 339,* 324-28, 1992), and a nonapeptide fragment of IL-2 (Zav'yalov *et al., Immunol. Lett. 31, 285-88,* 1992) all inhibit interleukin-2 dependent T lymphocyte proliferation; however, they are known to have many other effects. For example, cyclosporin is used as a immuno-suppressant after organ transplantation. While these agents may represent alternatives to steroids in the treatment of asthmatics, they inhibit interleukin-2 dependent T lymphocyte proliferation and potentially critical immune functions associated with homeostasis. Other treatments that block the release or activity of mediators of bronchochonstriction, such as cromones or anti-leukotrienes, have recently been introduced for the treatment of mild asthma, but they are expensive and not effective in all patients and it is unclear whether they have any effect on the chronic changes associated with asthmatic inflammation. What is needed in the art is the identification of a treatment that can act in pathways critical to the development of asthma that both blocks the episodic attacks of the disorder and preferentially dampens the hyperactive allergic immune response without immunocompromising the patient.

### Potential relevance of P2Y-like GPCR to asthma.

Extracellular nucleotides induce a wide variety of responses in many cell types, including muscle contraction and relaxation, vasodilation, neurotransmission, platelet aggregation, ion transport regulation, and cell growth. The effects are exerted through P2 receptors, which are classified into two main families: P2X receptors which are ligand-gated ion channels, and P2Y receptors which are G protein-coupled receptors. Twelve distinct P2Y family members have been cloned to date in various species, at least one of which is known to bind a non-nucleotide, namely P2Y₇ whose ligand is LTB₄. The nuclotide-binding P2Y receptors can be further subdivided into three groups according to ligand specificity: P2Ys activated by adenine nucleotides, P2Ys activated by uridine nucleotides, and P2Ys activated by both adenine and uridine nucleotides.

The human P2Y-like GPCR of the invention is a new P2Y-like seven-transmembrane-domain molecule that has highest homology to P2Y₁. It was originally found in a search for P2Y homologs in genomic sequence databases. Only one EST has been reported to date for this gene, from a cDNA library derived from normal human epithelium. Our own expression profiling of this gene shows that it is expressed highest in the trachea, salivary glands, and kidneys, and less so in fetal brain, colon, placenta, and lung.

Although human P2Y-like GPCR is closest in homology to P2Y1, which binds adenine nucleotides (ATP and ADP), it also has significant homology to P2Y₂ and P2Y₄, which bind both A and U nucleotides, to P2Y₃, which binds U nucleotides, and to leukotriene receptors, which bind LTB₄, LTC₄, and LTD₄. Therefore, although the likely range of ligands that human P2Y-like GPCR can bind is relatively limited, the true ligand of this receptor will have to be determined empirically.

In studies of airway epithelia, both ATP and UTP have been found to equipotently regulate epithelial electrolyte and water transport, trigger mucin secretion, and increase ciliary beat frequency. In the trachea, nucleotides can induce tracheal gland serous cells, which are responsible for the secretion of antibacterial and antiproteolytic proteins, to produce secretory leukocyte proteinase inhibitor and to increase chloride transport. Studies in a mouse knockout of the P2Y₂ receptor show that it is the dominant extracellular nucleotide receptor in airway epithelium, but that other nucleotide receptors exist that function similarly in the respiratory tract.

Our expression profiling studies of human P2Y-like GPCR show that it appears to be expressed highly in tissues of the upper respiratory tract. Its high expression in the salivary glands and trachea may indicate that it plays a role in exocrine secretion, which in the airways has mainly a protective role. In asthma, however, overproduction of mucin contributes to the viscid mucus plugs that occlude asthmatic airways. Submucosal glands in the large airways of asthmatics also frequently show evidence of hyperplasia, which may somehow be due to overstimulation by external mediators.

It is therefore unclear at this point what effect agonists or antagonists of the human P2Y-like GPCR receptor would have in asthmatics. Agonists may beneficially increase protective protein secretion, increase ciliary beat rate, and relax smooth muscle, while antagonists may slow mucus production and glandular hyperplasia.

### EXAMPLE 1

### Detection of P2Y1-like GPCR activity

The polynucleotide of SEQ ID NO: 1 is inserted into the expression vector pCEV4 and the expression vector pCEV4-P2Y1-like GPCR polypeptide obtained is transfected into human embryonic kidney 293 cells. These cells are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4 °C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of the added radioligand, are added to 96-well polypropylene microtiter plates containing ¹²⁵I-labeled ligand or test compound, non-labeled peptides, and binding buffer to a final volume of 250 µl.

In equilibrium saturation binding assays, membrane preparations are incubated in the presence of increasing concentrations (0.1 nM to 4 nM) of ¹²⁵I-labeled ligand or test compound (specific activity 2200 Ci/mmol). The binding affinities of different test compounds are determined in equilibrium competition binding assays, using 0.1 nM ¹²⁵I-peptide in the presence of twelve different concentrations of each test compound.

Binding reaction mixtures are incubated for one hour at 30 °C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. It is shown that the polypeptide of SEQ ID NO: 2 has a P2Y1-like GPCR activity.

### EXAMPLE 2

### Expression of recombinant human P2Y1-like GPCR

The *Pichia pastoris* expression vector pPICZB (Invitrogen, San Diego, CA) is used to produce large quantities of a human P2Y1-like GPCR polypeptides in yeast. The human P2Y1-like GPCR polypeptide-encoding DNA sequence is derived from the nucleotide sequence shown in SEQ ID NO:5. Before insertion into vector pPICZB the DNA sequence is modified by well known methods in such a way that it contains at its 5'-end an initiation codon and at its 3'-end an enterokinase cleavage site, a His6 reporter tag and a termination codon. Moreover, at both termini recognition sequences for restriction endonucleases are added and after digestion of the multiple cloning site of pPICZ B with the corresponding restriction enzymes the modified polypeptide encoding DNA sequence is ligated into pPICZB. This expression vector is designed for inducible expression in Pichia pastoris, expression is driven by a yeast promoter. The resulting pPICZ/md-His6 vector is used to transform the yeast.

The yeast are cultivated under usual conditions in 5 liter shake flasks and the recombinantly produced protein isolated from the culture by affinity chromatography (Ni-NTA-Resin) in the presence of 8 M urea. The bound polypeptide is eluted with buffer, pH 3.5, and neutralized. Separation of the P2Y1-like GPCR polypeptide from the His6 reporter tag is accomplished by site-specific proteolysis using enterokinase (Invitrogen, San Diego, CA) according to manufacturer's instructions. Purified human P2Y1-like GPCR polypeptide is obtained.

### EXAMPLE 3

### Radioligand binding assays

Human embryonic kidney 293 cells transfected with a polynucleotide which expresses human P2Y1-like GPCR are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4 °C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of the added radioligand, are added to 96-well polypropylene microtiter plates containing ¹²⁵I-labeled ligand or test compound, non-labeled peptides, and binding buffer to a final volume of 250 µl.

In equilibrium saturation binding assays, membrane preparations are incubated in the presence of increasing concentrations (0.1 nM to 4 nM) of ¹²⁵I-labeled ligand or test compound (specific activity 2200 Ci/mmol). The binding affinities of different test compounds are determined in equilibrium competition binding assays, using 0.1 nM ¹²⁵I-peptide in the presence of twelve different concentrations of each test compound.

Binding reaction mixtures are incubated for one hour at 30 °C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human P2Y1-like GPCR polypeptide.

### EXAMPLE 4

### Effect of a test compound on human P2Y1-like GPCR-mediated cyclic AMP formation

Receptor-mediated inhibition of cAMP formation can be assayed in host cells which express human P2Y1-like GPCR. Cells are plated in 96-well plates and incubated in Dulbecco's phosphate buffered saline (PBS) supplemented with 10 mM HEPES, 5 mM theophylline, 2 µg/ml aprotinin, 0.5 mg/ml leupeptin, and 10 µg/ml phosphoramidon for 20 minutes at 37 °C in 5% CO₂. A test compound is added and incubated for an additional 10 minutes at 37 °C. The medium is aspirated, and the reaction is stopped by the addition of 100 mM HCl. The plates are stored at 4 °C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

### EXAMPLE 5

### Effect of a test compound on the mobilization of intracellular calcium

Intracellular free calcium concentration can be measured by microspectrofluorometry using the fluorescent indicator dye Fura-2/AM (Bush *et al., J. Neurochem. 57,* 562-74, 1991). Stably transfected cells are seeded onto a 35 mm culture dish containing a glass coverslip insert. Cells are washed with HBS , incubated with a test compound, and loaded with 100 µl of Fura-2/AM (10 µM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

### EXAMPLE 6

### Effect of a test compound on phosphoinositide metabolism

Cells which stably express human P2Y1-like GPCR cDNA are plated in 96-well plates and grown to confluence. The day before the assay, the growth medium is changed to 100 µl of medium containing 1% serum and 0.5 µCi ³H-myinositol. The plates are incubated overnight in a CO₂ incubator (5% CO₂ at 37 °C). Immediately before the assay, the medium is removed and replaced by 200 µl of PBS containing 10 mM LiCl, and the cells are equilibrated with the new medium for 20 minutes. During this interval, cells also are equilibrated with antagonist, added as a 10 µl aliquot of a 20-fold concentrated solution in PBS.

The ³H-inositol phosphate accumulation from inositol phospholipid metabolism is started by adding 10 µl of a solution containing a test compound. To the first well 10 µl are added to measure basal accumulation. Eleven different concentrations of test compound are assayed in the following 11 wells of each plate row. All assays are performed in duplicate by repeating the same additions in two consecutive plate rows.

The plates are incubated in a CO₂ incubator for one hour. The reaction is terminated by adding 15 µl of 50% v/v trichloroacetic acid (TCA), followed by a 40 minute incubation at 4 °C. After neutralizing TCA with 40 µl of 1 M Tris, the content of the wells is transferred to a Multiscreen HV filter plate (Millipore) containing Dowex AG1-X8 (200-400 mesh, formate form). The filter plates are prepared by adding 200 µl of Dowex AG1-X8 suspension (50% v/v, water:resin) to each well. The filter plates are placed on a vacuum manifold to wash or elute the resin bed. Each well is washed 2 times with 200 µl of water, followed by 2 x 200 µl of 5 mM sodium tetraborate/60 mM ammonium formate.

The ³H-IPs are eluted into empty 96-well plates with 200 µl of 1.2 M ammonium formate/0.1 formic acid. The content of the wells is added to 3 ml of scintillation cocktail, and radioactivity is determined by liquid scintillation counting.

### EXAMPLE 7

### Receptor Binding Methods

Standard Binding Assays. Binding assays are carried out in a binding buffer containing 50 mM HEPES, pH 7.4, 0.5% BSA, and 5 mM MgCl₂. The standard assay for radioligand (*e.g*., ¹²⁵I- test compound) binding to membrane fragments comprising P2Y1-like GPCR polypeptides is carried out as follows in 96 well microtiter plates (*e.g*., Dynatech Immulon II Removawell plates). Radioligand is diluted in binding buffer+ PMSF/Baci to the desired cpm per 50 µl, then 50 µl aliquots are added to the wells. For non-specific binding samples, 5 µl of 40 µM cold ligand also is added per well. Binding is initiated by adding 150 µl per well of membrane diluted to the desired concentration (10-30 µg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 µl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used.
1. Competitive radioligand binding assays with a concentration range of cold ligand vs. ¹²⁵ I-labeled ligand are carried out as described above with one modification. All dilutions of ligands being assayed are made in 40X PMSF/Baci to a concentration 40X the final concentration in the assay. Samples of peptide (5 µl each) are then added per microtiter well. Membranes and radioligand are diluted in binding buffer without protease inhibitors. Radioligand is added and mixed with cold ligand, and then binding is initiated by addition of membranes.
2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.
3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

### EXAMPLE 8

### Chemical Cross-Linking of Radioligand to Receptor

After a radioligand binding step as described above, membrane pellets are resuspended in 200 µl per microtiter plate well of ice-cold binding buffer without BSA. Then 5 µl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and DuPont image intensifier screens.

### EXAMPLE 9

### Membrane Solubilization

Membrane solubilization is carried out in buffer containing 25 mM Tris , pH 8, 10% glycerol (w/v) and 0.2 mM CaCl₂ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

### EXAMPLE 10

### Assay of Solubilized Receptors

After binding of ¹²⁵I ligands and solubilization of the membranes with detergent, the intact R:L complex can be assayed by four different methods. All are carried out on ice or in a cold room at 4-10 °C).
1. Column chromatography (Knuhtsen *et al*., *Biochem*. *J*. *254,* 641-647, 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free ¹²⁵I ligands, is considered non-bound.
2. Polyethyleneglycol precipitation (Cuatrecasas, *Proc. Natl. Acad. Sci. USA 69,* 318-322, 1972). For a 100 µl sample of solubilized membranes in a 12 x 75 mm polypropylene tube, 0.5 ml of 1% (w/v) bovine gamma globulin (Sigma) in 0.1 M sodium phosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : ¹²⁵ I-ligand complex is determined by gamma counting of the filters.
3. GFB/PEI filter binding (Bruns *et al., Analytical Biochem. 132,* 74-81, 1983). Whatman GF/B glass fiber filters are soaked in 0.3% polyethyleneimine (PEI, Sigma) for 3 hours. Samples of solubilized membranes (25-100 µl) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor : ¹²⁵ I-ligand complex adsorbed to filters are determined by gamma counting.
4. Charcoal/Dextran (Paul and Said, *Peptides 7[Suppl. 1]*, 147-149, 1986). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4 °C. until use. To measure R:L complex, 4 parts by volume of charcoal/dextran suspension are added to 1 part by volume of solubilized membrane. The samples are mixed and held on ice for 2 minutes and then centrifuged for 2 minutes at 11,000 x g in a Beckman microfuge. Free radioligand is adsorbed charcoal/dextran and is discarded with the pellet. Receptor : ¹²⁵ I-ligand complexes remain in the supernatant and are determined by gamma counting.

### EXAMPLE 11

### Receptor Purification

Binding of biotinyl-receptor to GH₄ C1 membranes is carried out as described above. Incubations are for 1 hour at room temperature. In the standard purification protocol, the binding incubations contain 10 nM Bio-S29. ¹²⁵I ligand is added as a tracer at levels of 5,000-100,000 cpm per mg of membrane protein. Control incubations contain 10 µM cold ligand to saturate the receptor with non-biotinylated ligand.

Solubilization of receptor:ligand complex also is carried out as described above, with 0.15% deoxycholate:lysolecithin in solubilization buffer containing 0.2 mM MgCl₂, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10 °C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with solubilization buffer+0.1 mM EDTA+0.1 mM EGTA+0.1 mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the binding step, the resin is pelleted by centrifugation, the supernatant is removed and saved, and the resin is washed 3 times (about 2 minutes each) in buffer containing 50 mM HEPES, pH 8, 5 mM MgCl₂, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90 °C.

### EXAMPLE 12

### Identification of test compounds that bind to P2Y1-like GPCR polypeptides

Purified P2Y1-like GPCR polypeptides comprising a glutathione-S-transferase protein and absorbed onto glutathione-derivatized wells of 96-well microtiter plates are contacted with test compounds from a small molecule library at pH 7.0 in a physiological buffer solution. P2Y1-like GPCR polypeptides comprise an amino acid sequence shown in SEQ ID NO:2. The test compounds comprise a fluorescent tag. The samples are incubated for 5 minutes to one hour. Control samples are incubated in the absence of a test compound.

The buffer solution containing the test compounds is washed from the wells. Binding of a test compound to a P2Y1-like GPCR polypeptide is detected by fluorescence measurements of the contents of the wells. A test compound which increases the fluorescence in a well by at least 15% relative to fluorescence of a well in which a test compound was not incubated is identified as a compound which binds to a P2Y1-like GPCR polypeptide.

### EXAMPLE 13

### Tissue-specific expression of P2Y-Like GPCR

As a first step to establishing a role for P2Y-like GPCR in the pathogenesis of COPD, expression profiling of the gene was done using real-time quantitative PCR with RNA samples from human respiratory tissues and inflammatory cells relevant to COPD. The panel consisted of total RNA samples lung (adult and fetal), trachea, freshly isolated alveolar type II cells, cultured human bronchial epithelial cells, cultured small airway epithelial cells, cultured bronchial sooth muscle cells, cultured H441 cells (Clara-like), freshly isolated neutrophils and monocytes, and cultured monocytes (macrophage-like). Expression of P2Y-like GPCR also was evaluated in a range of human tissues using total RNA panels obtained from Clontech Laboratories, UK, Ltd.. The tissues were adrenal gland, bone marrow, brain, colon, heart, kidney, liver, lung, mammary gland, pancreas, prostate, salivary gland, skeletal muscle, small intesting, spleen, stomach, testis, thymus, trachea, thyroid, and uterus.

*Real-time quantitative PCR.* Expression profiling of the target gene was performed using real-time quantitative PCR, a development of the kinetic analysis of PCR first described in Higuchi *et al., BioTechnology 10,* 413-17, 1992, and Higuchi *et al., BioTechnology 11,* 1026-30, 1993. The principle is that at any given cycle within the exponential phase of PCR, the amount of product is proportional to the initial number of template copies.

PCR amplification is performed in the presence of an oligonucleotide probe (TaqMan probe) that is complementary to the target sequence and labeled with a fluorescent reporter dye and a quencher dye. During the extension phase of PCR, the probe is cleaved by the 5'-3' endonuclease activity of Taq DNA polymerase, releasing the fluorophore from the effect of the quenching dye (Holland *et al., Proc. Natl*. *Acad. Sci. U.S.A. 88*, 7276-80, 1991). Because the fluorescence emission increases in direct proportion to the amount of the specific amplified product, the exponential growth phase of PCR product can be detected and used to determine the initial template concentration (Heid *et al., Genome Res. 6,* 986-94, 1996, and Gibson *et al., Genome Res. 6*, 995-1001, 1996).

Real-time quantitative PCR was done using an ABI Prism 7700 Sequence Detector. The C_{T} value generated for each reaciton was used to determine the initial template concentration (copy number) by interpolation from a universal standard curve. The level of expression of the target gene in each sample was calculated relative to the sample with the lowest expression of the gene.

*RNA extraction and cDNA preparation.* Total RNA from each of the respiratory tissues and inflammatory cell types listed above were isolated using Qiagen's RNeasy system according to the manufacturer's protocol (Crawley, West Sussex, UK). The concentration of purified RNA was determined using a RiboGreen RNA quantitation kit (Molecular Probes Europe, The Netherlands). For the preparation of cDNA, 1 µg of total RNA was reverse transcribed in a final volume of 20 µl, using 200 U of SUPERSCRIPT™ RNase H⁻ Reverse Transcriptase (Life Technologies, Paisley, UK), 10 mM dithiothreitol, 0.5 mM of each dNTP and 5 µM random hexamers (Applied Biosystems, Warrington, Cheshire, UK) according to the manufacturer's protocol.

*TaqMan quantitative analysis.* Specific primers and probe were designed according to the recommendations of PE Applied Biosystems. The probe was labeled at the 5' end with FAM (6-carboxyfluorescein). Quantification PCR was performed with 5 ng of reverse transcribed RNA from each sample. Each determination is done in duplicate.

The assay reaction mix was as follows: 1X final TaqMan Universal PCR Master Mix (from 2X stock) (PE Applied Biosystems, CA); 900 nM forward primer; 900 nM reverse primer; 200 nM probe; 5 ng cDNA; and water to 25 µl.

Each of the following steps were carried out once: pre PCR, 2 minutes at 50° C, and 10 minutes at 95°C. The following steps are carried out 40 times: denaturation, 15 seconds at 95°C, annealing/extension, 1 minute at 60°C.

All experiments were performed using an ABI Prism 7700 Sequence Detector (PE Applied Biosystems, CA). At the end of the run, fluorescence data acquired during PCR were processed as described in the ABI Prism 7700 user's manual to achieve better background subtraction as well as signal linearity with the starting target quantity.

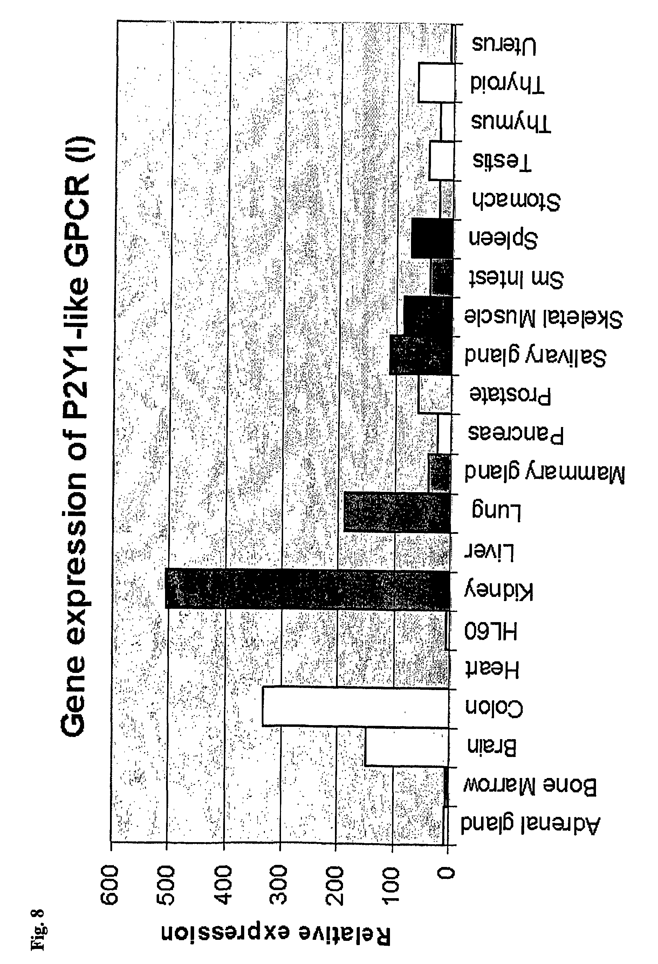

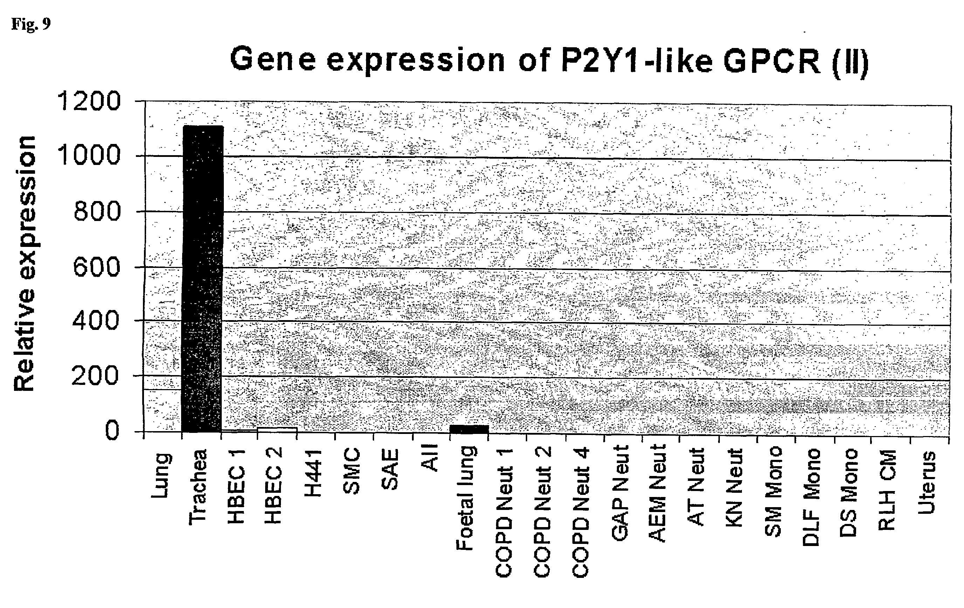

Tables 1 and 2 show the results of expression profiling for P2Y-like GPCR using the indicated cell and tissue samples. For Table 1, the cells are defined as follows: HBEC, cultured human bronchial epithelial cells; H441, a Clara-like cell line; SAE, cultured small airway epithelial cells; SMC, cultured airway smooth muscle cells; AII, freshly isolated human alveolar type II cells; Neut, freshly isolated circulating neutrophils; Mono, freshly isolated monocytes; and CM, cultured monocytes. Other letters identify the donor. The results are shown graphically in FIGS. 8 and 9.

**Table 1.**

| **Tissue** | **Relative expression** |
|---|---|
| Lung | 151.5354699 |
| Trachea | 1107.743218 |
| HBEC 1 | 4.685060463 |
| HBEC 2 | 12.9520377 |
| H441 | 1.202387631 |
| SMC | 1.41842932 |
| SAE | 1.749411592 |
| AII | 1 |
| Foetal lung | 26.72970095 |
| COPD Neut 1 | 0.897587922 |
| COPD Neut 2 | 0.76087585 |
| COPD Neut 4 | 0 |
| GAP Neut | 1.694694812 |
| AEM Neut | 1.331087791 |
| AT Neut | 0 |
| KN Neut | 0.987369255 |
| SM Mono | 0.891902539 |
| DLF Mono | 0.926568646 |
| DS Mono | 1.257087188 |
| RLH CM | 0 |
| Uterus | 0 |

**Table 2.**

| **Tissue** | **Relative expression** |
|---|---|
| Adrenal gland | 9.189455584 |
| Bone Marrow | 5.852208398 |
| Brain | 149.621596 |
| Colon | 333.2508632 |
| Heart | 1.093054592 |
| HL60 | 4.424597636 |
| Kidney | 506.9221673 |
| Liver | 1 |
| Lung | 190.4931147 |
| Mammary gland | 40.91893135 |
| Pancreas | 22.80291052 |
| Prostate | 58.4105483 |
| Salivary gland | 109.5837815 |
| Skeletal Muscle | 84.98460946 |
| Sm Intest | 40.14618513 |
| Spleen | 71.58421767 |
| Stomach | 23.68924846 |
| Testis | 41.70655162 |
| Thymus | 22.94834038 |
| Thyroid | 63.44162273 |
| Uterus | 4.125836209 |

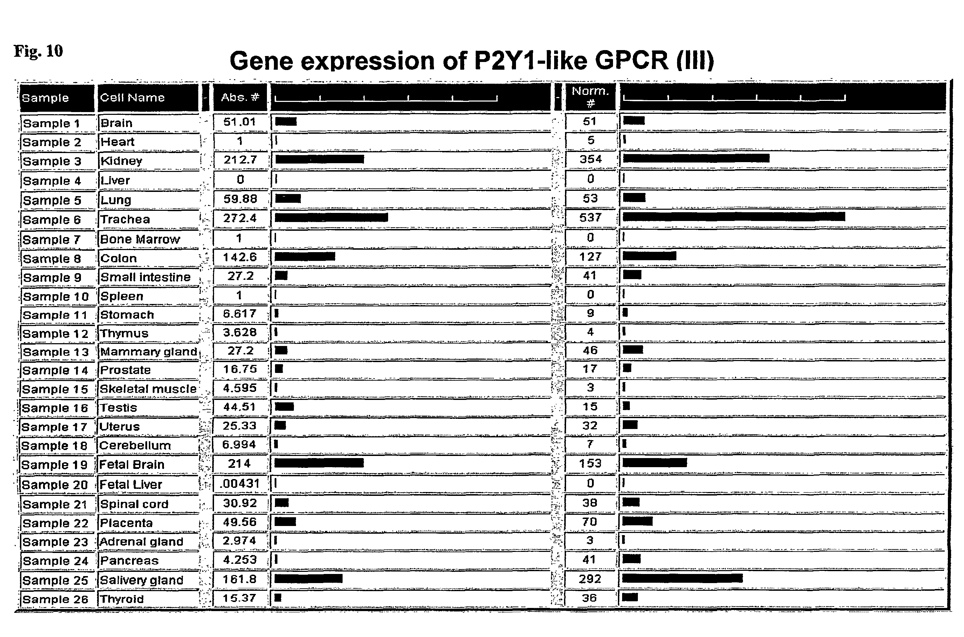

Furthermore, a specific gene expression of P2Y1-like GPCR (III) is shown in Fig. 10. In this experiment a polymerase chain reaction was carried out using oligonucleotide primers LBRI119cds-L2 (ttcggatcgaatctcgcctgct) and LBRI119cds-R2 (tgcttgctcaaggttcccgctta) and measurements of the intensity of emitted light were taken following each cycle of the reaction when the reaction had reached a temperature of 82 degrees C.

### Potential relevance of P2Y1-like GPCR to asthma.

Extracellular nucleotides induce a wide variety of responses in many cell types, including muscle contraction and relaxation, vasodilation, neurotransmission, platelet aggregation, ion transport regulation, and cell growth. The effects are exerted through P2 receptors, which are classified into two main families: P2X receptors which are ligand-gated ion channels, and P2Y receptors which are G protein-coupled receptors. Twelve distinct P2Y family members have been cloned to date in various species, at least one of which is known to bind a non-nucleotide, namely P2Y₇ whose ligand is LTB₄. The nuclotide-binding P2Y receptors can be further subdivided into three groups according to ligand specificity: P2Ys activated by adenine nucleotides, P2Ys activated by uridine nucleotides, and P2Ys activated by both adenine and uridine nucleotides.

P2Y1-like GPCR is a new P2Y-like seven-transmembrane-domain molecule that has highest homology to P2Y₁. It was originally found in a search for P2Y homologs in genomic sequence databases. Only one EST has been reported to date for this gene, from a cDNA library derived from normal human epithelium. Our own expression profiling of this gene shows that it is expressed highest in the trachea, salivary glands, and kidneys, and less so in fetal brain, colon, placenta, and lung.

Although P2Y1-like GPCR is closest in homology to P2Y1, which binds adenine nucleotides (ATP and ADP), it also has significant homology to P2Y₂ and P2Y₄, which bind both A and U nucleotides, to P2Y₃, which binds U nucleotides, and to leukotriene receptors, which bind LTB₄, LTC₄, and LTD₄. Therefore, although the likely range of ligands that P2Y1-like GPCR can bind is relatively limited, the true ligand of this receptor will have to be determined empirically.

In studies of airway epithelia, both ATP and UTP have been found to equipotently regulate epithelial electrolyte and water transport, trigger mucin secretion, and increase ciliary beat frequency. In the trachea, nucleotides can induce tracheal gland serous cells, which are responsible for the secretion of antibacterial and antiproteolytic proteins, to produce secretory leukocyte proteinase inhibitor and to increase chloride transport. Studies in a mouse knockout of the P2Y₂ receptor show that it is the dominant extracellular nucleotide receptor in airway epithelium, but that other nucleotide receptors exist that function similarly in the respiratory tract.

Our expression profiling studies of P2Y1-like GPCR show that it appears to be expressed highly in tissues of the upper respiratory tract. Its high expression in the salivary glands and trachea may indicate that it plays a role in exocrine secretion, which in the airways has mainly a protective role. In asthma, however, overproduction of mucin contributes to the viscid mucus plugs that occlude asthmatic airways. Submucosal glands in the large airways of asthmatics also frequently show evidence of hyperplasia, which may somehow be due to overstimulation by external mediators.

It is therefore unclear at this point what effect agonists or antagonists of the P2Y1-like GPCR P2Y receptor would have in asthmatics. Agonists may beneficially increase protective protein secretion, increase ciliary beat rate, and relax smooth muscle, while antagonists may slow mucus production and glandular hyperplasia.

### References.

Nandanan E, Jang SY, Moro S, Kim HO, Siddiqui MA, Russ P, Marquez VE, Busson R, Herdewijn

P, Harden TK, Boyer JL, Jacobson KA. Synthesis, biological activity, and molecular modeling of ribose-modified deoxyadenosine bisphosphate analogues as P2Y(1) receptor ligands. *J Med Chem*. 2000 Mar 9;43(5):829-42.

Major DT, Halbfinger E, Fischer B. Molecular recognition of modified adenine nucleotides by the P2Y(1)-receptor. 2. A computational approach. *J Med Chem. 1999 Dec 30;42(26):5338-47*.

Halbfinger E, Major DT, Ritzmann M, Ub1 J, Reiser G, Boyer JL, Harden KT, Fischer B. Molecular recognition of modified adenine nucleotides by the P2Y(1)-receptor. 1. A synthetic, biochemical, and NMR approach. *J Med Chem*. 1999 Dec 30;42(26):5325-37.

Moro S, Hoffmann C, Jacobson KA. Role of the extracellular loops of G protein-coupled receptors in ligand recognition: a molecular modeling study of the human P2Y1 receptor. *Biochemistry.* 1999 Mar 23;38(12):3498-507.

Moro S, Guo D, Camaioni E, Boyer JL, Harden TK, Jacobson KA. Human P2Y1 receptor: molecular modeling and site-directed mutagenesis as tools to identify agonist and antagonist recognition sites. *J Med Chem.* 1998 Apr 23;41(9):1456-66.

Webb TE, Simon J, Barnard EA. Regional distribution of [35S]2'-deoxy 5'-O-(1-thio) ATP binding sites and the P2Y1 messenger RNA within the chick brain. *Neuroscience.* 1998 Jun;84(3):825-37.

Homolya L, Watt WC, Lazarowski ER, Koller BH, Boucher RC. Nucleotide-regulated calcium signaling in lung fibroblasts and epithelial cells from normal and P2Y(2) receptor (-/-) mice. *J Biol Chem*. 1999 Sep 10;274(37):26454-60.

Leon C, Hechler B, Vial C, Leray C, Cazenave JP, Gachet C. The P2Y1 receptor is an ADP receptor antagonized by ATP and expressed in platelets and megakaryoblastic cells. *FEBS Lett*. 1997 Feb 10;403(1):26-30.

Dehaye JP, Moran A, Marino A. Purines, a new class of agonists in salivary glands? *Arch Oral Biol.* 1999 May;44 Suppl 1:S39-43.

King BF, Townsend-Nicholson A, Bumstock G. Metabotropic receptors for ATP and UTP: exploring the correspondence between native and recombinant nucleotide receptors. *Trends Pharmacol Sci.* 1998 Dec;19(12):506-14.

Saleh A, Figarella C, Kammouni W, Marchand-Pinatel S, Lazdunski A, Tubul A, Brun P, Merten MD. Pseudomonas aeruginosa quorum-sensing signal molecule N-(3-oxododecanoyl)-L-homoserine lactone inhibits expression of P2Y receptors in cystic fibrosis tracheal gland cells. *Infect Immun*. 1999 Oct;67(10):5076-82.

Cressman VL, Lazarowski E, Homolya L, Boucher RC, Koller BH, Grubb BR. Effect of loss of P2Y(2) receptor gene expression on nucleotide regulation of murine epithelial Cl(-) transport. *J Biol Chem*. 1999 Sep 10;274(37):26461-8.

## Claims

1. Use of a screening method, said method comprising the steps of
(i) contacting a test compound with a polypeptide comprising an amino acid sequence which is at least 90% identical to the amino acid sequence of SEQ ID NO:2, and
(ii) detecting binding of said test compound to a polypeptide of (i);
for the identification of compounds useful in the treatment of asthma.

2. Use of a screening method, said method comprising the steps of
(i) contacting a test compound with a polypeptide comprising an amino acid sequence which is at least 90% identical to the amino acid sequence of SEQ ID NO:2, and
(ii) detecting an activity of the polypeptide of (i);
for the identification of compounds useful in the treatment of asthma.

3. Use of Claim 1 or 2, wherein the contacting step is in, or at the surface of, a cell.

## Patentansprüche

1. Verwendung eines Screening-Verfahrens, wobei besagtes Verfahren die folgenden Schritte umfasst:
(i) In-Kontakt-Bringen einer Testverbindung mit einem Polypeptid, welches eine Aminosäuresequenz umfasst, welche zu mindestens 90 % identisch ist mit der Aminosäuresequenz von SEQ ID NO:2, und
(ii) Detektieren der Bindung der besagten Testverbindung an ein Polypeptid des Schritts (i);
zur Identifikation von Verbindungen, welche für die Behandlung von Asthma verwendbar sind.

2. Verwendung eines Screening-Verfahrens, wobei besagtes Verfahren die folgenden Schritte umfasst:
(i) In-Kontakt-Bringen einer Testverbindung mit einem Polypeptid, welches eine Aminosäuresequenz umfasst, welche zu mindestens 90 % identisch ist mit der Aminosäuresequenz von SEQ ID NO:2, und
(ii) Detektieren einer Wirksamkeit des Polypeptids des Schritts (i);
zur Identifikation von Verbindungen, welche für die Behandlung von Asthma verwendbar sind.

3. Verwendung von Anspruch 1 oder 2, wobei der Schritt des In-Kontakt-Bringens in einer Zelle oder auf der Oberfläche einer Zelle stattfindet.

## Revendications

1. Utilisation d'un procédé de criblage, ledit procédé comprenant les étapes consistant à :
(i) mettre en contact un composé d'essai avec un polypeptide comprenant une séquence d'acides aminés qui présente au moins 90 % d'identité avec la séquence d'acides aminés de SEQ ID NO : 2, et
(ii) détecter la liaison dudit composé d'essai à un polypeptide de (i) ;
pour l'identification de composés utiles dans le traitement de l'asthme.

2. Utilisation d'un procédé de criblage, ledit procédé comprenant les étapes consistant à :
(i) mettre en contact un composé d'essai avec un polypeptide comprenant une séquence d'acides aminés qui présente au moins 90 % d'identité avec la séquence d'acides aminés de SEQ ID NO : 2, et
(ii) détecter une activité du polypeptide de (i) ;
pour l'identification de composés utiles dans le traitement de l'asthme.

3. Utilisation selon la revendication 1 ou 2, dans laquelle l'étape de mise en contact s'effectue dans une cellule ou à la surface d'une cellule.


### SEQUENCE LISTING

<110> Bayer AG
<120> REGULATION OF HUMAN P2Y1-LIKE G PROTEIN-COUPLED RECEPTOR
<130> LIO131 Foreign Countries
<150> US 60/224,989
   <151> 2000-08-14
<160> 6
<170> PatentIn version 3.1
<210> 1
   <211> 593
   <212> DNA
   <213> Homo sapiens
<400> 1
   tggcttttga gtcagaaaaa tgcgagtgtg gatcccagct aagcccctag atgtgttacc      60
   agagacgagc cgcaaaacat ttctcagtct tagtctcttg taaaatagag gtaataagaa     120
   acacttttca gtattttgtg acatgtagaa gtaagtgatg gtggcatgca tcacacttgg     180
   ttaatagtag gtcctgttgt taagtctcta atggcgatac cctatggctt ctccaaatgg     240
   tgaccttgcc aaattgtttt ccaaagcgac atgtggcttt tttctcccaa tccctcattt     300
   taactctcat ggtaatttaa cttttatatt tttattagat gcatttagta acttgcctca     360
   tagtcatttt cttggaaatt caatttcttc tccacagggt ctcttttgag attaaagaga     420
   gagaagtggc aaatttagga tgttagaata attttcattt aaaagtagat ccttgttttt     480
   attaccctat cattaatgtt ttctgttttc ctttatcagc gagttactgc tcatttgatt     540
   catattgcca aactgaactc tcttgttttc ttgcaagatg aaaggagaca acc            593
<210> 2
   <211> 337
   <212> PRT
   <213> Homo sapiens
<400> 2
   Met Asn Glu Pro Leu Asp Tyr Leu Ala Asn Ala Ser Asp Phe Pro Asp
   1 5 10 15
   Tyr Ala Ala Ala Phe Gly Asn Cys Thr Asp Glu Asn Ile Pro Leu Lys
   20 25 30
   Met His Tyr Leu Pro Val Ile Tyr Gly Ile Ile Phe Leu Val Gly Phe
   35 40 45
   Pro Gly Asn Ala Val Val Ile Ser Thr Tyr Ile Phe Lys Met Arg Pro
   50 55 60
   Trp Lys Ser Ser Thr Ile Ile Met Leu Asn Leu Ala Cys Thr Asp Leu
   65 70 75 80
   Leu Tyr Leu Thr Ser Leu Pro Phe Leu Ile His Tyr Tyr Ala Ser Gly
   85 90 95
   Glu Asn Trp Ile Phe Gly Asp Phe Met Cys Lys Phe Ile Arg Phe Ser
   100 105 110
   Phe His Phe Asn Leu Tyr Ser Ser Ile Leu Phe Leu Thr Cys Phe Ser
   115 120 125
   Ile Phe Arg Tyr Cys Val Ile Ile His Pro Met Ser Cys Phe Ser Ile
   130 135 140
   His Lys Thr Arg Cys Ala Val Val Ala Cys Ala Val Val Trp Ile Ile
   145 150 155 160
   Ser Leu Val Ala Val Ile Pro Met Thr Phe Leu Ile Thr Ser Thr Asn
   165 170 175
   Arg Thr Asn Arg Ser Ala Cys Leu Asp Leu Thr Ser Ser Asp Glu Leu
   180 185 190
   Asn Thr Ile Lys Trp Tyr Asn Leu Ile Leu Thr Ala Thr Thr Phe Cys
   195 200 205
   Leu Pro Leu Val Ile Val Thr Leu Cys Tyr Thr Thr Ile Ile His Thr
   210 215 220
   Leu Thr His Gly Leu Gln Thr Asp Ser Cys Leu Lys Gln Lys Ala Arg
   225 230 235 240
   Arg Leu Thr Ile Leu Leu Leu Leu Ala Phe Tyr Val Cys Phe Leu Pro
   245 250 255
   Phe His Ile Leu Arg Val Ile Arg Ile Glu Ser Arg Leu Leu Ser Ile
   260 265 270
   Ser Cys Ser Ile Glu Asn Gln Ile His Glu Ala Tyr Ile Val Ser Arg
   275 280 285
   Pro Leu Ala Ala Leu Asn Thr Phe Gly Asn Leu Leu Leu Tyr Val Val
   290 295 300
   Val Ser Asp Asn Phe Gln Gln Ala Val Cys Ser Thr Val Arg Cys Lys
   305 310 315 320
   Val Ser Gly Asn Leu Glu Gln Ala Lys Lys Ile Ser Tyr Ser Asn Asn
   325 330 335
   Pro
<210> 3
   <211> 673
   <212> DNA
   <213> Homo sapiens
<400> 3
   aatatttcat ttacttaacc aaaaacaaat acttgctgat actttaccta gcatcctaag 60
   atgttcagga tgtctccctc aatggaactc ctggtaaata ctgtgtattc aagtaatcat 120
   gtgccaaagc cagggcagag cttctagttc tttgcaatcc ctttattgag ctcctccact 180
   ggggagatat aagaatggga tgcatgtata tcagcaaagt attcagacat agtattacaa 240
   gctattggaa ctcagaggca tcttagagaa catctgttcc caccaactta ctatatatac 300
   acggaaacca atttcttacc cttgccctag attgctcagt aaatttgtgc caagatagga 360
   gaaaaccaat cttttcactc atcatttcat gcttctctgc actctgggcc tatttgtatt 420
   gaaccattag acaattcaaa ccactacttg tatctttctt aatatttatt ttttacatct 480
   cagagctcta caatttgttt ccttcaagct taactttgag attataaaac tgggtttagc    540
   cagttctgta tattacttca agccagtaag ataccctgga aataatccaa ggacgtccat    600
   gcaaatagtt gaaattagta cctgcaatat atttggagta ttatgtcttt attgttgtta 660
   aaaagttttt att 673
<210> 4
   <211> 2245
   <212> DNA
   <213> Homo sapiens
<220>
   <221> misc_feature
   <222> (19174) .. (1916)
   <223> start codon
<220>
   <221> promoter
   <222> (1112)..(1117)
   <223> TATA box
<400> 4
   aacaagactg ccacagtcgt atcctggctc acccctaact agcggtttga ctttgagcac 60
   atcactgcct cctccgtgcc tcagtttcct cttctgttga atggggatga tcacagcact 120
   acctcaccat ttgttgtgag gattaaatag attcaaatat gtaagacact tacagtaatg    180
   ctttgtacac agaaggcact attatttttt attaatccct atttttcctt cccaacttca    240
   tctcccaata tcccataacc atgctgattt ccttacaact cccccagacc tcctggacta    300
   agtgagattt ggaagagtat actcagggca gtcaagaagg actgattctg ctaatttagg    360
   atttgtcaag ttgggttagt ttgaaatata cctctgtact cctctcagga aatgctggat    420
   agtgtagatt tagtatcaga taatagtaat aatggtggca gactctgatg tttcctcctt    480
   cccttgtgat ctcagtagtc ttgtaccatt catacatcaa atgacttagc aactaacaat    540
   cacgtaatat tttttaagct tttcttatgt gcccagtagt gagccaagag ttttttttcc    600
   tatttttaac atactattga cagctccatg aggtgggttt atattttgtt aaaataattt    660
   gccatgtcca cttccccagt agactgtgac agtggcatat acatctgtgt gtgttattca    720
   gtttgtatct ctaggacttg acagaggacc caatacagag tgggtgctcc agaaatgtgg    780
   acttggatgc atttcaccaa cagctattta catccaaata ataagctaat tataactctt    840
   cttctagatg tacttttctc attgcaaaaa tcaccgtcaa aatttttaaa aacactaata    900
   agcaagaaga aggaagttaa aattgcactc cttccagccc tagagagctt aatcaacttg    960
   cttaggaaca cgctactaaa tggctaatct gagcaccaaa atctggccat ctggcttcag   1020
   ggctcagcct cctaatggcc gcattatacc attgatcccg ccccatccac cccaacacac   1080
   acactcccct gcacaaacaa attatgttag ctattaaaag gaattggaag agtggaaatg   1140
   tgcaattaaa tctaccacaa atacaatgtt gcaatacgtg caatctatat ttttaaaatt   1200
   tacagtattc agcattttcc tgtcttcaca cttgttccct ccgttattta cggcaatcat   1260
   gacgaaaatc aacaaaatac agattgagag gcagcactga gaggtaatgt agaaagggaa   1320
   tggcttttga gtcagaaaaa tgcgagtgtg gatcccagct aagcccctag atgtgttacc   1380
   agagacgagc cgcaaaacat ttctcagtct tagtctcttg taaaatagag gtaataagaa   1440
   acacttttca gtattttgtg acatgtagaa gtaagtgatg gtggcatgca tcacacttgg   1500
   ttaatagtag gtcctgttgt taagtctcta atggcgatac cctatggctt ctccaaatgg   1560
   tgaccttgcc aaattgtttt ccaaagcgac atgtggcttt tttctcccaa tccctcattt   1620
   taactctcat ggtaatttaa cttttatatt tttattagat gcatttagta acttgcctca   1680
   tagtcatttt cttggaaatt caatttcttc tccacagggt ctcttttgag attaaagaga   1740
   gagaagtggc aaatttagga tgttagaata attttcattt aaaagtagat ccttgttttt   1800
   attaccctat cattaatgtt ttctgttttc ctttatcagc gagttactgc tcatttgatt   1860
   catattgcca aactgaactc tcttgttttc ttgcaagatg aaaggagaca accatgaatg   1920
   agccactaga ctatttagca aatgcttctg atttccccga ttatgcagct gcttttggaa   1980
   attgcactga tgaaaacatc ccactcaaga tgcactacct ccctgttatt tatggcatta 2040
   tcttcctcgt gggatttcca ggcaatgcag tagtgatatc cacttacatt ttcaaaatga 2100
   gaccttggaa gagcagcacc atcattatgc tgaacctggc ctgcacagat ctgctgtatc 2160
   tgaccagcct ccccttcctg attcactact atgccagtgg cgaaaactgg atctttggag 2220
   atttcatgtg taagtttatc cgctt 2245
<210> 5
   <211> 1014
   <212> DNA
   <213> Homo sapiens
<400> 5
   atgaatgagc cactagacta tttagcaaat gcttctgatt tccccgatta tgcagctgct 60
   tttggaaatt gcactgatga aaacatccca ctcaagatgc actacctccc tgttatttat 120
   ggcattatct tcctcgtggg atttccaggc aatgcagtag tgatatccac ttacattttc 180
   aaaatgagac cttggaagag cagcaccatc attatgctga acctggcctg cacagatctg 240
   ctgtatctga ccagcctccc cttcctgatt cactactatg ccagtggcga aaactggatc 300
   tttggagatt tcatgtgtaa gtttatccgc ttcagcttcc atttcaacct gtatagcagc 360
   atcctcttcc tcacctgttt cagcatcttc cgctactgtg tgatcattca cccaatgagc 420
   tgcttttcca ttcacaaaac tcgatgtgca gttgtagcct gtgctgtggt gtggatcatt 480
   tcactggtag ctgtcattcc gatgaccttc ttgatcacat caaccaacag gaccaacaga 540
   tcagcctgtc tcgacctcac cagttcggat gaactcaata ctattaagtg gtacaacctg 600
   attttgactg caactacttt ctgcctcccc ttggtgatag tgacactttg ctataccacg 660
   attatccaca ctctgaccca tggactgcaa actgacagct gccttaagca gaaagcacga 720
   aggctaacca ttctgctact ccttgcattt tacgtatgtt ttttaccctt ccatatcttg 780
   agggtcattc ggatcgaatc tcgcctgctt tcaatcagtt gttccattga gaatcagatc 840
   catgaagctt acatcgtttc tagaccatta gctgctctga acacctttgg taacctgtta 900
   ctatatgtgg tggtcagcga caactttcag caggctgtct gctcaacagt gagatgcaaa 960
   gtaagcggga accttgagca agcaaagaaa attagttact caaacaaccc ttga 1014
<210> 6
   <211> 373
   <212> PRT
   <213> Homo sapiens
<400> 6
   Met Thr Glu Val Leu Trp Pro Ala Val Pro Asn Gly Thr Asp Ala Ala
   1               5                   10                  15
   Phe Leu Ala Gly Pro Gly Ser Ser Trp Gly Asn Ser Thr Val Ala Ser
               20                  25                  30
   Thr Ala Ala Val Ser Ser Ser Phe Lys Cys Ala Leu Thr Lys Thr Gly
           35                  40                  45
   Phe Gln Phe Tyr Tyr Leu Pro Ala Val Tyr Ile Leu Val Phe Ile Ile
       50                  55                  60
   Gly Phe Leu Gly Asn Ser Val Ala Ile Trp Met Phe Val Phe His Met
   65                  70                  75                  80
   Lys Pro Trp Ser Gly Ile Ser Val Tyr Met Phe Asn Leu Ala Leu Ala
                   85                  90                  95
   Asp Phe Leu Tyr Val Leu Thr Leu Pro Ala Leu Ile Phe Tyr Tyr Phe
               100                 105                 110
   Asn Lys Thr Asp Trp Ile Phe Gly Asp Ala Met Cys Lys Leu Gln Arg
           115                 120                 125
   Phe Ile Phe His Val Asn Leu Tyr Gly Ser Ile Leu Phe Leu Thr Cys
       130                 135                 140
   Ile Ser Ala His Arg Tyr Ser Gly Val Val Tyr Pro Leu Lys Ser Leu
   145                 150                 155                 160
   Gly Arg Leu Lys Lys Lys Asn Ala Ile Cys Ile Ser Val Leu Val Trp
                   165                 170                 175
   Leu Ile Val Val Val Ala Ile Ser Pro Ile Leu Phe Tyr Ser Gly Thr
               180                 185                 190
   Gly Val Arg Lys Asn Lys Thr Ile Thr Cys Tyr Asp Thr Thr Ser Asp
           195                 200                 205
   Glu Tyr Leu Arg Ser Tyr Phe Ile Tyr Ser Met Cys Thr Thr Val Ala
       210                 215                 220
   Met Phe Cys Val Pro Leu Val Leu Ile Leu Gly Cys Tyr Gly Leu Ile
   225                 230                 235                 240
   Val Arg Ala Leu Ile Tyr Lys Asp Leu Asp Asn Ser Pro Leu Arg Arg
                   245                 250                 255
   Lys Ser Ile Tyr Leu Val Ile Ile Val Leu Thr Val Phe Ala Val Ser
               260                 265                 270
   Tyr Ile Pro Phe His Val Met Lys Thr Met Asn Leu Arg Ala Arg Leu
   275 280 285
   Asp Phe Gln Thr Pro Ala Met Cys Ala Phe Asn Asp Arg Val Tyr Ala
   290 295 300
   Thr Tyr Gln Val Thr Arg Gly Leu Ala Ser Leu Asn Ser Cys Val Asp
   305 310 315 320
   Pro Ile Leu Tyr Phe Leu Ala Gly Asp Thr Phe Arg Arg Arg Leu Ser
   325 330 335
   Arg Ala Thr Arg Lys Ala Ser Arg Arg Ser Glu Ala Asn Leu Gln Ser
   340 345 350
   Lys Ser Glu Asp Met Thr Leu Asn Ile Leu Pro Glu Phe Lys Gln Asn
   355 360 365
   Gly Asp Thr Ser Leu
   370